(19) Europäisches Patentamt / European Patent Office / Office européen des brevets

(11) **EP 4 365 310 A1**

(12) **EUROPEAN PATENT APPLICATION**
published in accordance with Art. 153(4) EPC

(43) Date of publication:
**08.05.2024   Bulletin 2024/19**

(21) Application number: **22811708.1**

(22) Date of filing: **30.05.2022**

(51) International Patent Classification (IPC):
***C12Q 1/6886*** *(2018.01)*

(52) Cooperative Patent Classification (CPC):
**C12Q 1/6886**

(86) International application number:
**PCT/KR2022/007695**

(87) International publication number:
**WO 2022/250517 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2021   KR 20210069428**

(71) Applicant: **Sysbiogen Co., Ltd.**
**Seongnam-si, Gyeonggi-do 13558 (KR)**

(72) Inventors:
• **CHOI, Kwan Yong**
  **Yongin-si, Gyeonggi-do 16809 (KR)**
• **KIM, Jong Min**
  **Pohang-si, Gyeongsangbuk-do 37673 (KR)**
• **HEO, Tae Yang**
  **Pohang-si, Gyeongsangbuk-do 37664 (KR)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **BIOMARKER FOR DIAGNOSING PROSTATE CANCER AND USE THEREOF**

(57)   The present invention relates to a biomarker for diagnosing prostate cancer and a use thereof, and, more particularly, to: a composition for diagnosing prostate cancer, comprising a formulation that can measure the expression level of a specific lncRNA, miRNA, and/or mRNA of a fusion gene, or a fragment thereof; a method for providing information for diagnosing prostate cancer, comprising a step for measuring the expression level of a biomarker; and a kit for diagnosing prostate cancer, comprising the composition for diagnosing prostate cancer. The present invention identifies, for the first time, a combination of a novel lncRNA, miRNA, and/or mRNA of a fusion gene abnormally expressed in prostate cancer in comparison to a normal tissue, and uses the combination as a biomarker, and, thus, can quickly and rapidly diagnose prostate cancer through a non-invasive method without a surgical procedure. Particularly, the lncRNA, miRNA, and mRNA of a fusion gene, of the present invention, for diagnosing prostate cancer are expressed in relatively large amounts in comparison to other genes in the urine of a subject and, thus, have the benefit of being able to be stably analyzed via qRT-PCR.

[FIG 1]

EP 4 365 310 A1

**Description**

[Technical Field]

The present invention relates to a biomarker for diagnosing prostate cancer, and a use thereof, and more particularly, to a composition for diagnosing prostate cancer, which includes an agent that can measure an expression level of specific lncRNA, miRNA, mRNA of a fusion gene, and/or a fragment thereof; a method of providing information for diagnosing prostate cancer, which includes measuring an expression level of the biomarker; and a kit for diagnosing prostate cancer, which includes the composition for diagnosing prostate cancer.

[Background Art]

The prostate is a walnut-sized male reproductive organ that is located just below the bladder and in front of the rectum, and serves to produce and store a portion of semen. The prostate is fixed above to the puboprostatic ligament on its front, adjacent to the bladder neck, that is, the area transitioning from the bladder to the urethra, and below by the urogenital diaphragm. Most cancers that occur in the prostate are adenocarcinomas (cancers of glandular cells) that arise from prostate cells. Tumor types may be distinguished according to the degree of differentiation of tumor tissue and the characteristics of cells.

Prostate cancer (PCa) is one of the most prevalent urinary tract tumors worldwide. In the United States, approximately 180,890 people were newly diagnosed with prostate cancer in 2016 alone, accounting for 10.7% of all new tumor diagnoses in the United States, and prostate cancer is the third most frequently occurring tumor after breast cancer and lung cancer. Based on statistics from 2009 to 2013, 129.4 out of 100,000 males worldwide had prostate cancer, and the mortality for prostate cancer reached 26,120 in 2016. In addition, prostate cancer is an uncommon disease before the age of 50, but its incidence increases rapidly after the age of 50. Recently, due to the increase in average life expectancy, the number of elderly men is rapidly increasing in Korea, and prostate cancer needs to be diagnosed early and continuously managed to prevent it from worsening, such as metastasis. Particularly, human prostate cancer appears to have a tendency of metastasis to bone, and it is known to inevitably progress from an androgen-dependent state to an androgen-resistant state, increasing the mortality of patients. Moreover, prostate cancer is a disease that recurs in approximately 25% of men who receive treatment for prostate cancer and requires additional treatment. Prostate cancer is currently the second leading cause of death of cancer among men in the United States (Jemal A et al., CA Cancer J Clin 2010;60:277-300), and early diagnosis and treatment for this are needed.

Conventional techniques for diagnosing prostate cancer include prostate-specific antigen (PSA) measurement and a biopsy. PSA measurement is a method that is most widely used in prostate cancer diagnosis and is for determining the risk of prostate cancer by measuring a level of specific antigens produced in prostate cells. PSA levels are higher in malignant prostate cancer, and most men without prostate cancer have levels of less than 4 ng/mL. However, if the measured PSA level is very high despite the absence of prostate cancer or is in the PSA gray zone (PSA 4 ng/mL or more and 10 ng/mL or less), a biopsy is required. A biopsy is a method of diagnosing the presence or absence of malignant tumors such as cancer, sarcoma, etc., in the corresponding organ of a patient through histopathological examination by taking tissue from an organ area suspected of having cancer using a needle. Such a biopsy test is very painful for patients, and the accuracy of prostate cancer diagnosis particularly in the PSA gray zone is less than 30%. That is, there are disadvantages in that approximately 70% of patients undergo a costly and painful rebiopsy due to the limitations in PSA measurement.

Accordingly, to overcome and replace unnecessary rebiopsy and the limitations in PSA measurement, there is a need to develop a prostate cancer-specific diagnostic method with high accuracy.

Under this background, to avoid pain during the test and reduce the burden of costs, the present invention identified a specific miRNA combination and a combination of lncRNA, miRNA, and/or mRNA (particularly, mRNA of a fusion gene) as prostate cancer-specific biomarkers from urine samples of patients diagnosed with prostate cancer and urine samples of a non-tumor control, and confirmed that the combination of biomarkers such as miRNAs, lncRNA, miRNA, and/or mRNAs of fusion genes are useful in the diagnosis of prostate cancer. Thus, the present invention was completed.

[Disclosure]

[Technical Problems]

Accordingly, the present invention is directed to providing a composition that can effectively diagnose prostate cancer.

The present invention is also directed to providing a method of providing information for effectively diagnosing prostate cancer using a biomarker.

The present invention is also directed to providing a kit for diagnosing prostate cancer, which includes the composition.

The present invention is also directed to providing a method of diagnosing prostate cancer using the composition or the kit for diagnosing prostate cancer including the same.

[Technical Solutions]

To achieve the purposes of the present invention, the present invention provides a composition for diagnosing prostate cancer, which includes an agent for measuring an mRNA expression level.

The miRNA may be at least one selected from the group consisting of hsa_miR_375, hsa_miR_27b_3p, hsa_miR_31_5p, hsa_miR_125b_5p, hsa_miR_146a_3p, hsa_miR_146a_5p, hsa_miR_17_3p, hsa_miR_200b_3p, hsa_miR_21_5p, hsa_miR_1185_2_3p, hsa_miR_141_5p, hsa_miR_222_3p, hsa_miR_24_3p, hsa_miR_30a_3p, hsa_miR_30a_5p, hsa_miR_30b_5p, and hsa_miR_30c_5p. Preferably, the miRNA includes hsa_miR_125b_5p, hsa_miR_17_3p, hsa_miR_141_5p, and hsa_miR_30c_5p.

The miRNA may include hsa_miR_125b_5p, hsa_miR_17_3p, hsa_miR_141_5p, and hsa_miR_30c_5p, and further include at least one selected from the group consisting of hsa_miR_21_5p, hsa_miR_24_3p, hsa_miR_30a_5p, hsa_miR_222_3p, hsa_miR_375, hsa_miR_27b_3p, hsa_miR_31_5p, hsa_miR_146a_3p, hsa_miR_146a_5p, hsa_miR_200b_3p, hsa_miR_1185_2_3p, hsa_miR_30a_3p, and hsa_miR_30b_5p.

In one embodiment, the composition may further include at least one selected from the group consisting of lncRNA and mRNA of a fusion gene, wherein the lncRNA may be at least one selected from PCA3 and MALAT1, and the mRNA of a fusion gene may be TMPRSS2:ERG.

In one embodiment, the composition is a composition for diagnosing prostate cancer, including an agent that can measure an expression level of the following combinations, which may be: (a) lncRNA and miRNA, (b) mRNA of a fusion gene and miRNA, or (c) lncRNA, miRNA, and mRNA of a fusion gene. Here, the lncRNA may be at least one selected from PCA3 and MALAT1, and the mRNA of a fusion gene may be TMPRSS2:ERG.

In one embodiment, the composition may include a primer, a probe, and/or an antibody, each of which specifically binds to the miRNA.

In one embodiment, the agent may include a primer, a probe, and/or an antibody, each of which specifically binds to the lncRNA, miRNA and/or mRNA of a fusion gene.

In addition, the present invention provides a method of providing information for diagnosing prostate cancer, which includes: measuring an expression level of the lncRNA, mRNA of a fusion gene, miRNA, or a combination thereof in a biological sample isolated from a subject; and comparing an expression level of the lncRNA, mRNA of a fusion gene, miRNA, or a combination thereof with an expression level of lncRNA, mRNA of a fusion gene, miRNA, or a combination thereof in a normal control sample.

In one embodiment, an expression level of the lncRNA, mRNA of a fusion gene, miRNA, or a combination thereof may be measured by reverse transcriptase polymerase chain reaction (RT-PCR), competitive RT-PCR, quantitative RT-PCR (qPCR), droplet digital PCR (ddPCR), sequencing, an RNase protection method, Northern blotting, and/or gene chip analysis.

In one embodiment, the measuring of an expression level may include: extracting the lncRNA, mRNA of a fusion gene, miRNA, or a combination thereof from a biological sample isolated from a subject; synthesizing cDNA from the extracted lncRNA, mRNA of a fusion gene, miRNA, or combination thereof; amplifying the cDNA using a primer pair specific for the synthesized cDNA; and detecting the amplified cDNA.

In one embodiment, the biological sample may be tissue, cells, urine, saliva, semen, whole blood, plasma, and/or serum. Preferably, the biological sample is urine.

In one embodiment, the comparing of an expression level of the lncRNA, mRNA of a fusion gene, miRNA, or a combination thereof may be performed using a machine learning algorithm selected from the group consisting of logistic regression (LR), a support vector machine (SVM), a random forest, and a multi-layer perceptron. Preferably, the comparing of an expression level of lncRNA, mRNA of a fusion gene, miRNA, or a combination thereof may be performed using LR.

In addition, the present invention provides a kit for diagnosing prostate cancer, including the composition for diagnosing prostate cancer.

In addition, the present invention provides a method of diagnosing prostate cancer using the composition for diagnosing prostate cancer or the kit for diagnosing prostate cancer including the same.

[Advantageous Effect]

The present invention first identified a new miRNA combination and a new combination of IncRNA, miRNA, and mRNA (particularly, mRNA of a fusion gene), which are abnormally expressed in prostate cancer compared to normal tissue. These combinations can be used as biomarkers to quickly and rapidly diagnose prostate cancer non-invasively without a surgical procedure. Particularly, since the miRNA combination and the combination of IncRNA, miRNA, and mRNA of a fusion gene for diagnosing prostate cancer of the present invention show relatively higher expression levels than other genes in urine of a subject, they can be stably analyzed through qPCR.

[Description of Drawings]

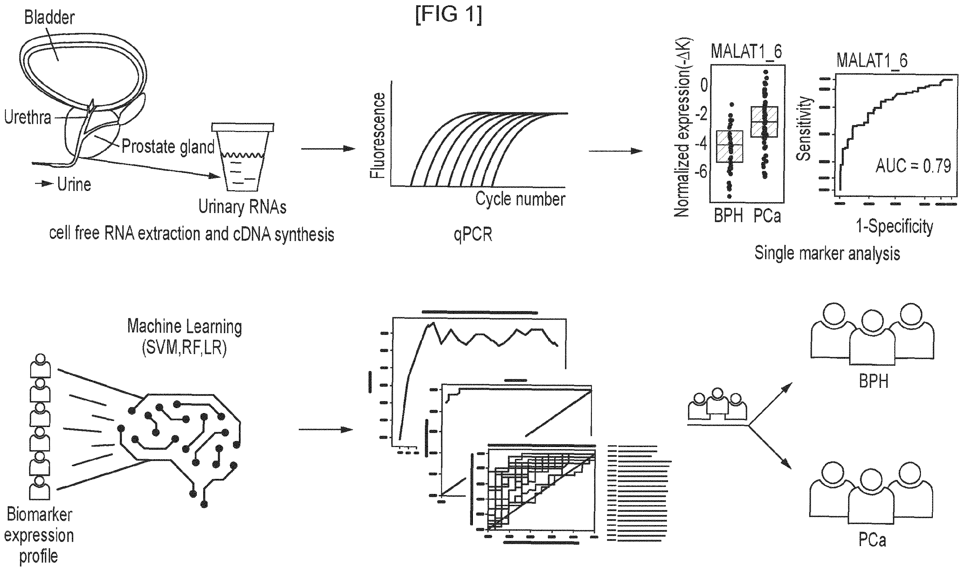

FIG. 1 shows an overview of machine learning-assisted multi-marker analysis for prostate cancer (PCa) screening using qPCR.

FIGS. 2 and 3 show results of single marker analysis: FIG. 2 is a box plot for the trend analysis of target gene expression; and FIG. 3 shows a receiver operating characteristic (ROC) curve and an area under the curve (AUC) for biomarker performance analysis.

FIG. 4 shows an optimal biomarker combination as an RFECV algorithm-applied result: (a) when 4 features are combined, a local maximum is formed; and (b) the rank of the biomarker used.

FIG. 5 shows machine learning process- and single biomarker-based ML classifier LOOCV scores.

FIG. 6 shows single biomarker-based machine learning evaluation.

FIG. 7 shows the multi-biomarker-based ML classifier evaluation for a four-miRNA combination (hsa_miR_125b_5p, hsa_miR_17_3p, hsa_miR_141_5p, and hsa_miR_30c_5p).

FIG. 8 shows the ML classifier evaluation and feature analysis for a four-miRNA combination (hsa_miR_125b_5p, hsa_miR_17_3p, hsa_miR_141_5p, and hsa_miR_30c_5p), and shows the result of machine learning-assisted multi-marker analysis.

FIG. 9 shows the multi-biomarker-based ML classifier evaluation for a six-biomarker combination (hsa_miR_125b_5p, hsa_miR_17_3p, hsa_miR_141_5p, hsa_miR_30c_5p, PCA3_4, and TMPRSS2:ERG Fusion_R3).

FIG. 10 shows the ML classifier evaluation and feature analysis for a six-biomarker combination (hsa_miR_125b_5p, hsa_miR_17_3p, hsa_miR_141_5p, hsa_miR_30c_5p, PCA3_4, and TMPRSS2:ERG Fusion_R3), which is the result of machine learning-assisted multi-marker analysis.

[Modes of the Invention]

The present invent ion relates to a biomarker for diagnosing prostate cancer.

The term "prostate cancer" used herein is a malignant tumor occurring in the prostate, and includes, but is not limited to, localized prostate cancer and advanced prostate cancer.

The term "diagnosis" used herein refers to confirmation of the presence or features of a pathological state. In the present invention, the diagnosis may be interpreted as confirming the onset or progression of prostate cancer.

The term "marker or diagnostic marker" used herein refers to a material that can diagnose prostate cancer by distinguishing a subject with prostate cancer from normal cells or a normal subject, and includes organic biomolecules such as a polypeptide, protein, or a nucleic acid (e.g., IncRNA, miRNA, or mRNA), a lipid, a glycolipid, a glycoprotein, or a sugar (a monosaccharide, a disaccharide, or an oligosaccharide), which shows an increase or decrease in cells or a subject, in which prostate cancer has developed or progressed, compared to normal cells, but the present invention is not limited thereto. Since a prostate cancer diagnostic marker may become an indicator for the onset and progression of prostate cancer, it may be used in the onset, progression, and diagnosis of prostate cancer.

For the purpose of the present invention, the prostate cancer diagnostic marker of the present invention is a fragment of IncRNA, miRNA, and/or mRNA of a fusion gene, which shows a difference in expression level specifically in prostate cancer cells, compared to normal cells or tissue cells.

More specifically, the IncRNA marker for diagnosing prostate cancer according to the present invention is at least one selected from PCA3 and MALAT1, and the fusion gene mRNA marker for diagnosing prostate cancer is TMPRSS2:ERG, and the miRNA marker for diagnosing prostate cancer is at least one selected from the group consisting of hsa_miR_375, hsa_miR_27b_3p, hsa_miR_31_5p, hsa_miR_125b_5p, hsa_miR_146a_3p, hsa_miR_146a_5p, hsa_miR_17_3p, hsa_miR_200b_3p, hsa_miR_21_5p, hsa_miR_1185_2_3p, hsa_miR_141_5p, hsa_miR_222_3p, hsa_miR_24_3p, hsa_miR_30a_3p, hsa_miR_30a_5p, hsa_miR_30b_5p, and hsa_miR_30c_5p, which are RNAs that increase or decrease in cells or a subject in which prostate cancer has developed or progressed. Among these markers, PCA3, TMPRSS2:ERG, and MALAT1 are increased in expression in the case of prostate cancer, but decreased in expression in other cases.

In one embodiment, particularly, two or more of the markers may be used in combination to diagnose a prostate cancer patient from a normal control and improve the ability to distinguish the progression of prostate cancer. Among these markers according to the present invention, the combination of lncRNA, mRNA and/or miRNA, which exhibits the optimal effect for such purposes, may be selected and used, and a suitable combination for the purpose may be selected by those of ordinary skill in the art.

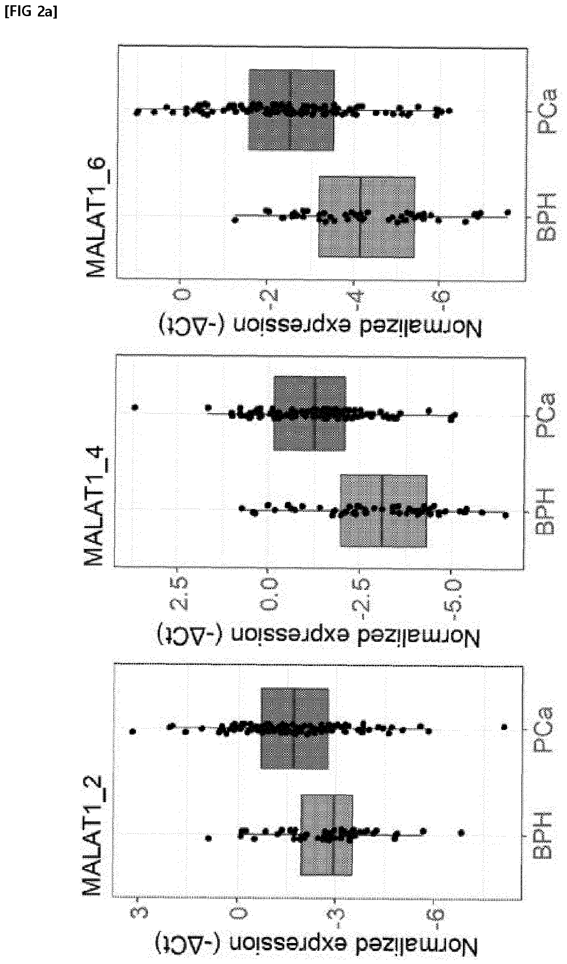

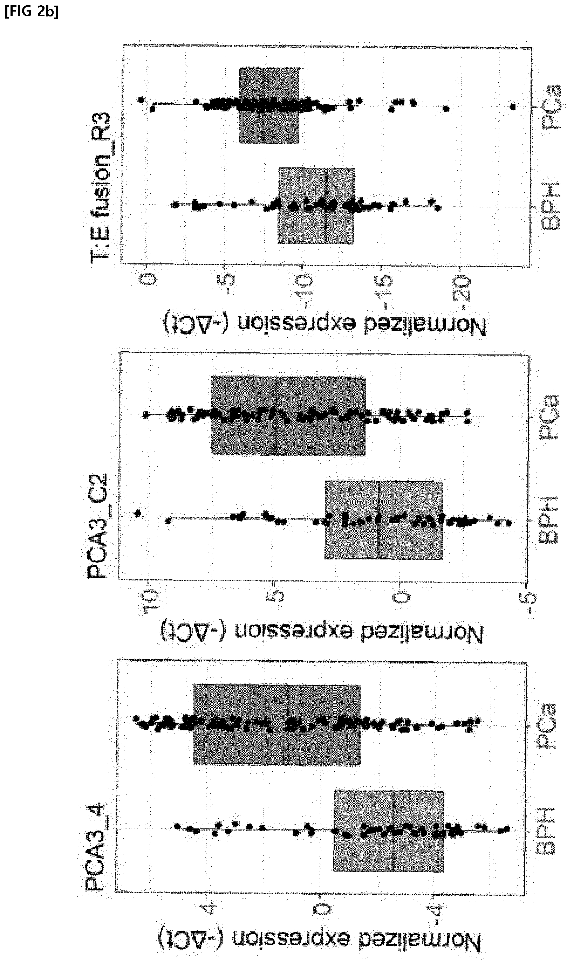

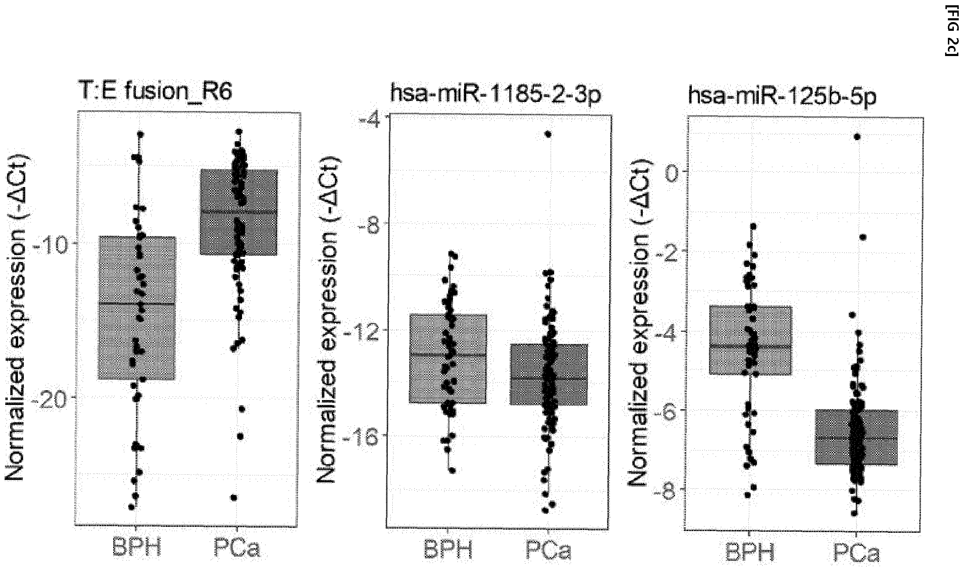

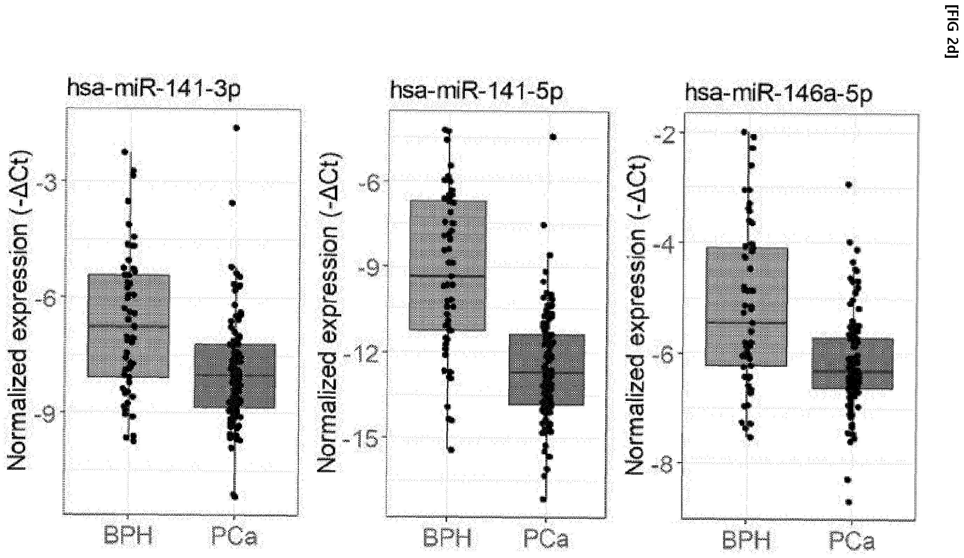

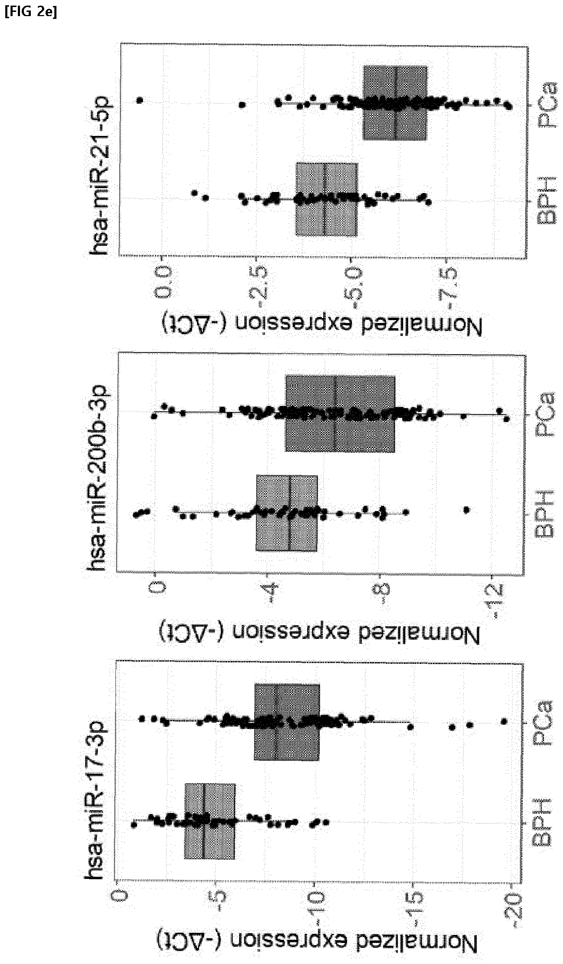

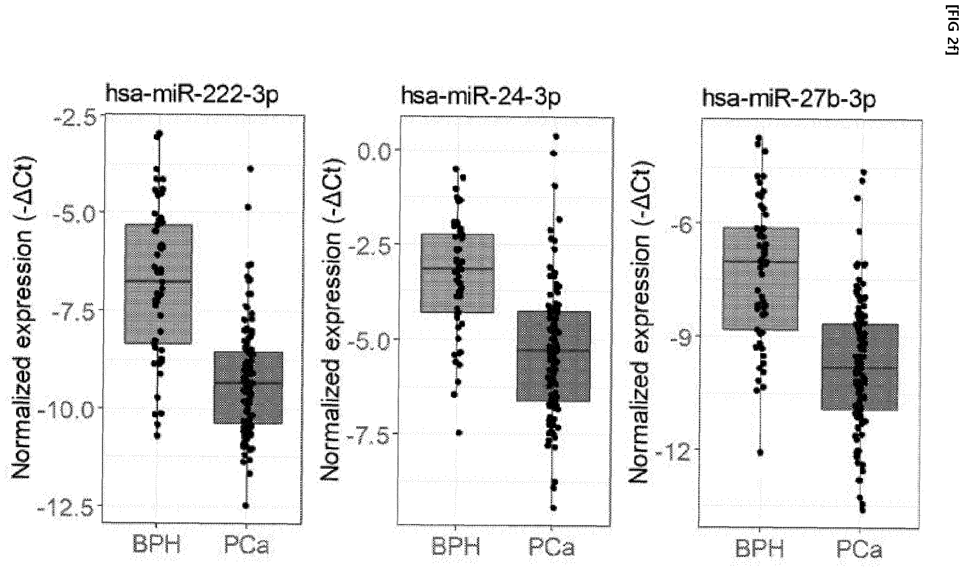

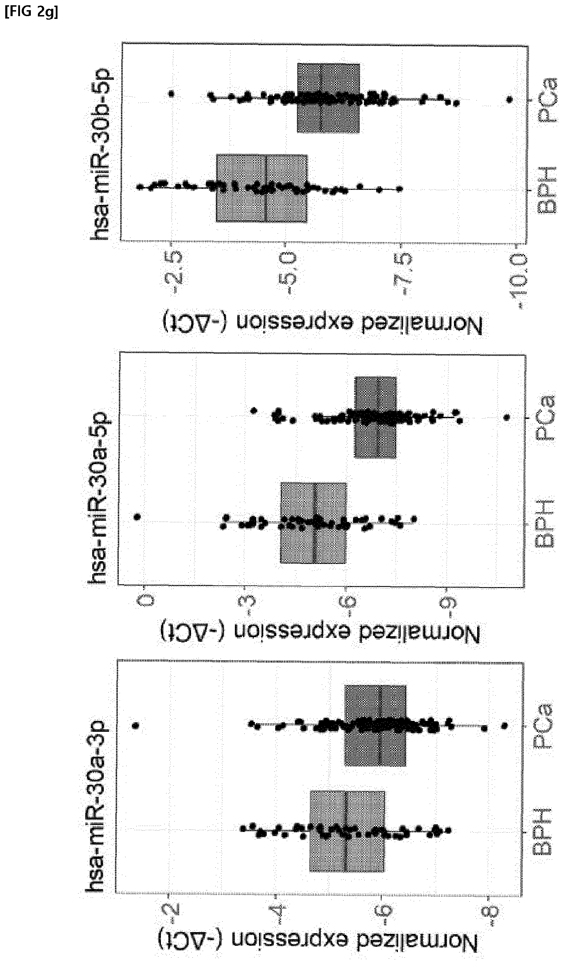

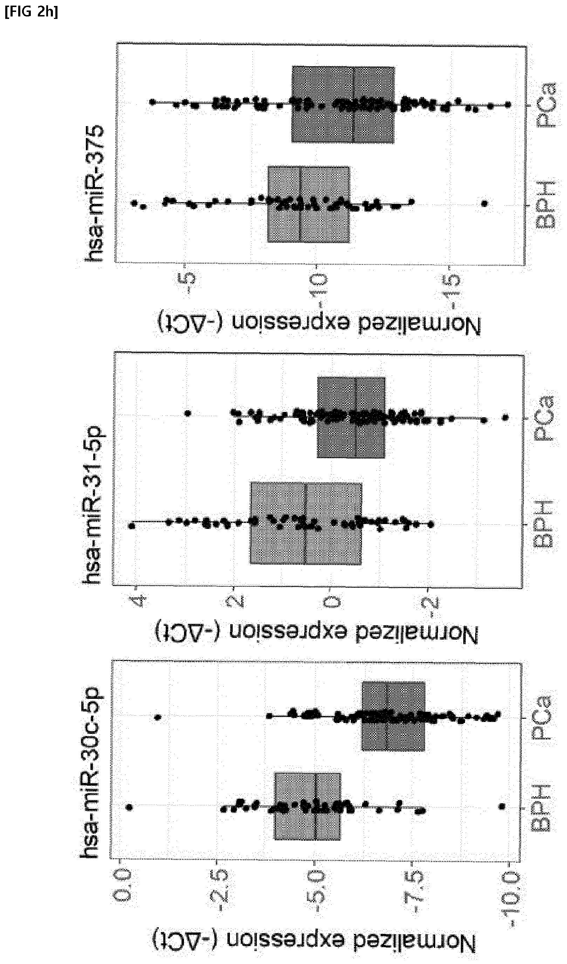

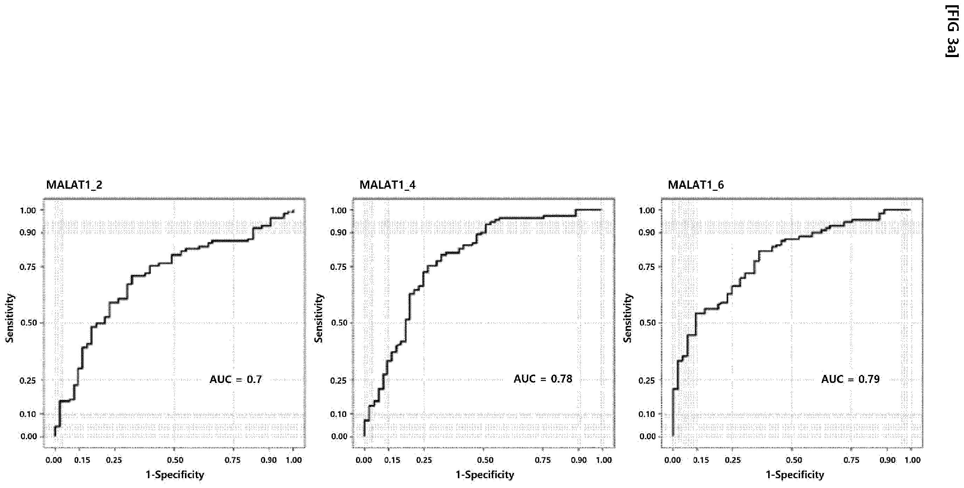

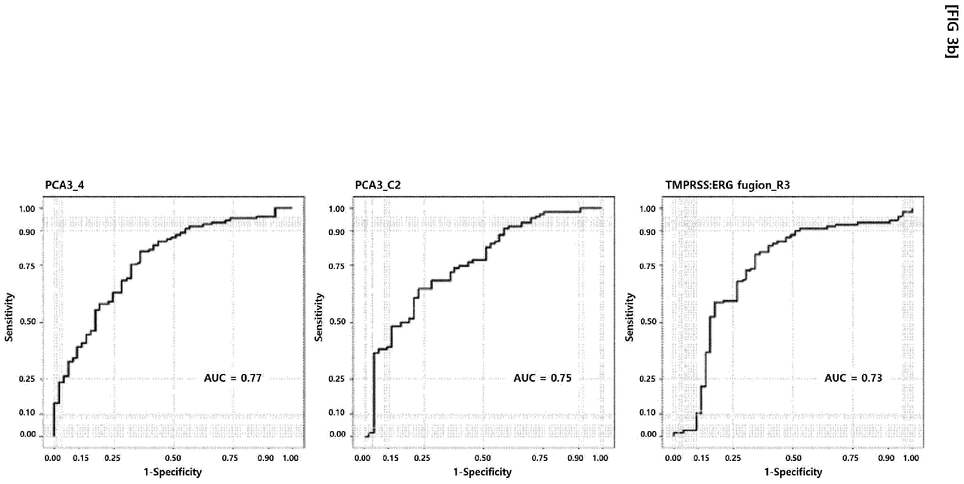

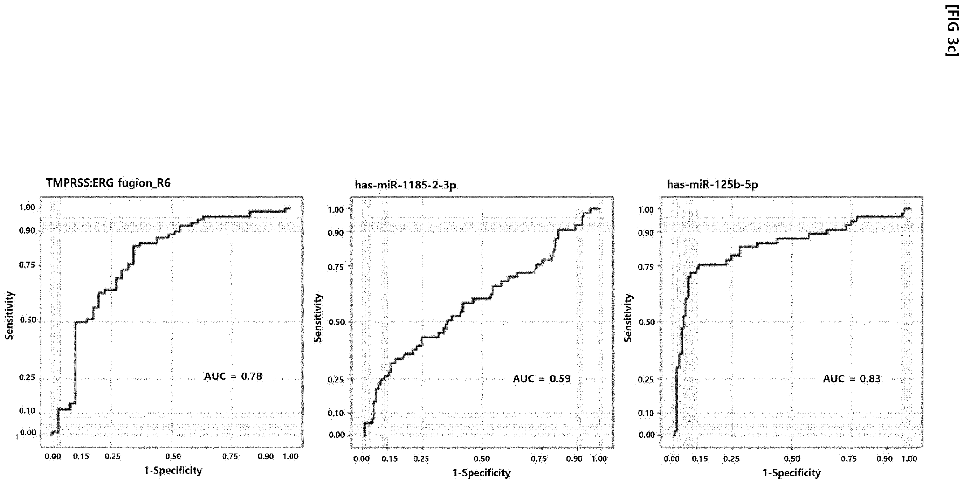

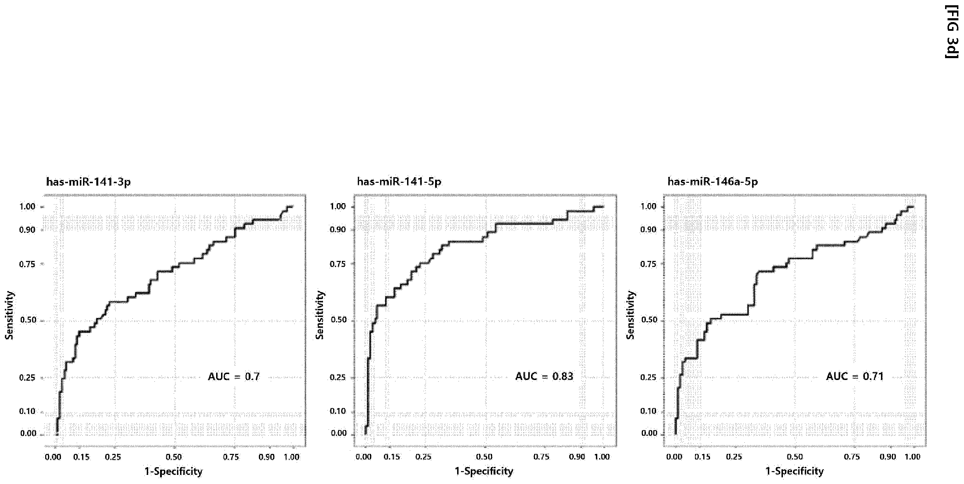

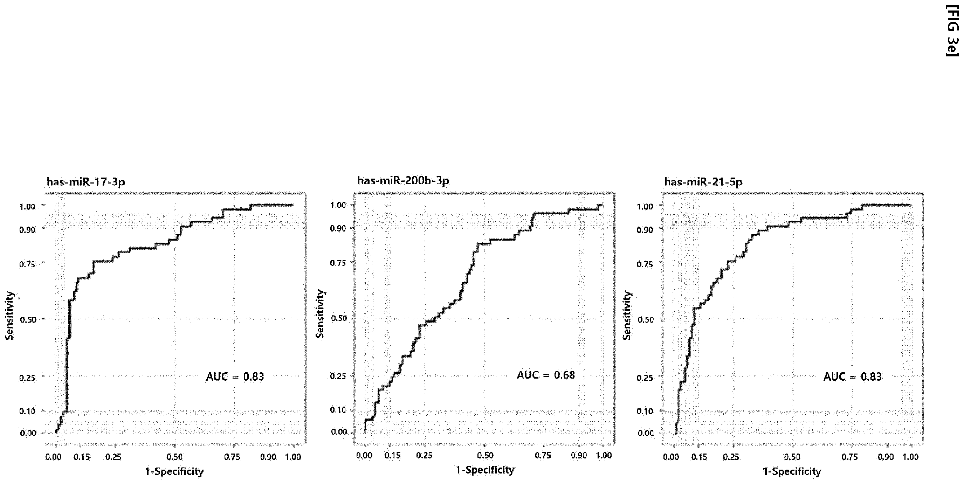

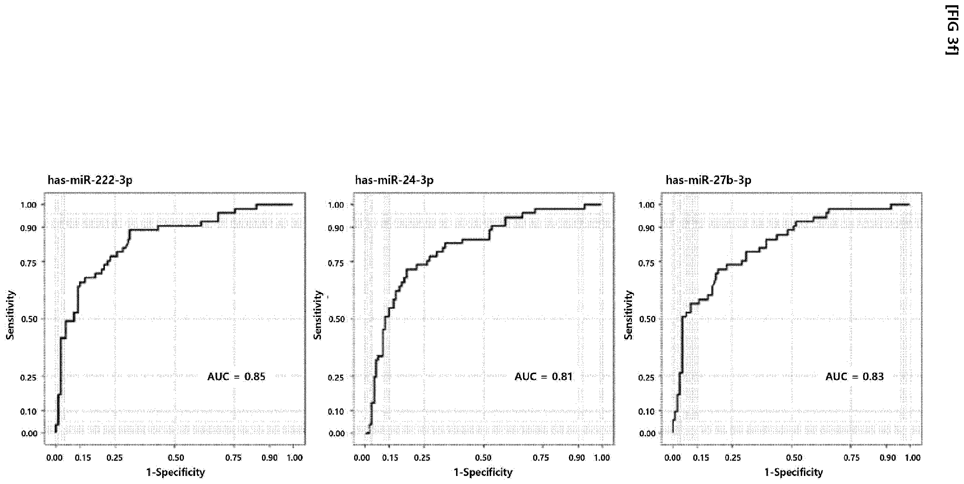

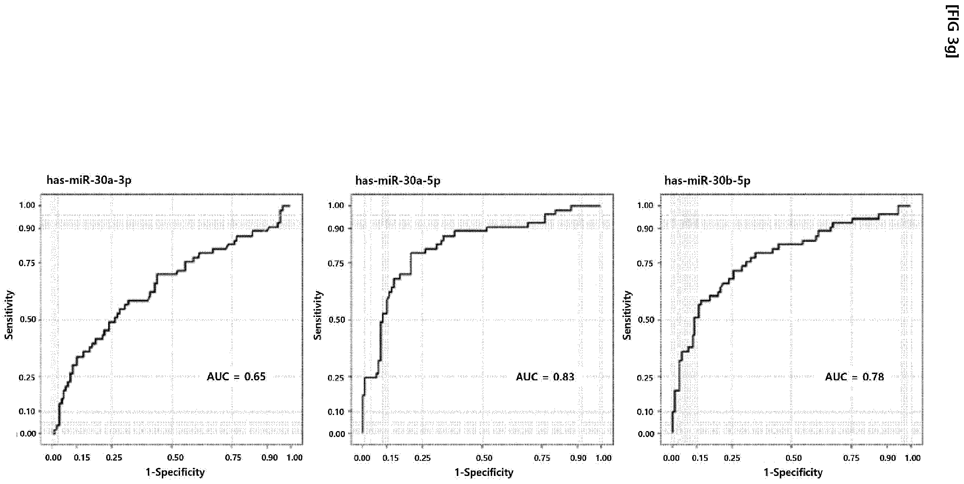

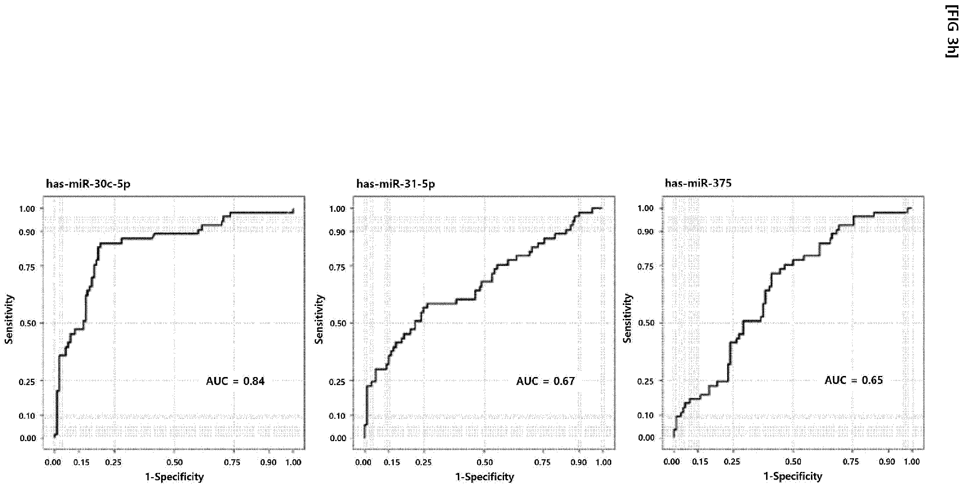

In one embodiment, the combination of the markers, such as lncRNA, mRNA and/or miRNA, may be selected by relative expression level analysis of markers according to a patient group and evaluation for individual marker performance by AUC values of an ROC curve. In detail, primers that have high AUC values or consistent patient group-dependent trends with existing references may be selected and then chosen by additionally performing multi-variable analysis. The individual data analysis result for each biomarker is disclosed in FIGS. 2 and 3.

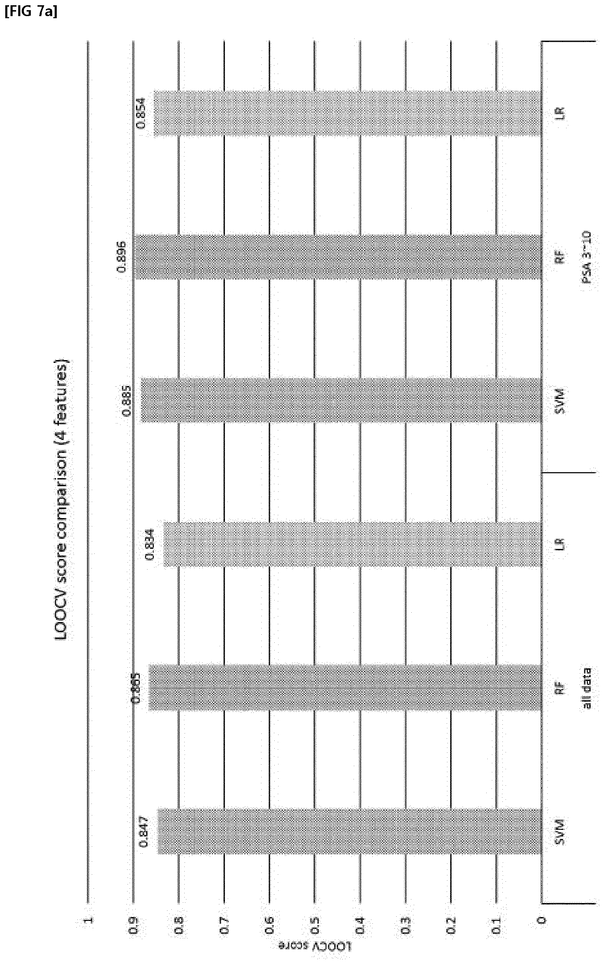

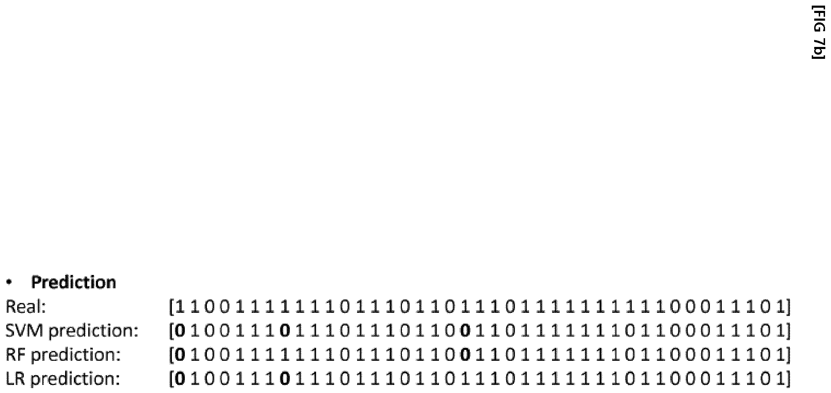

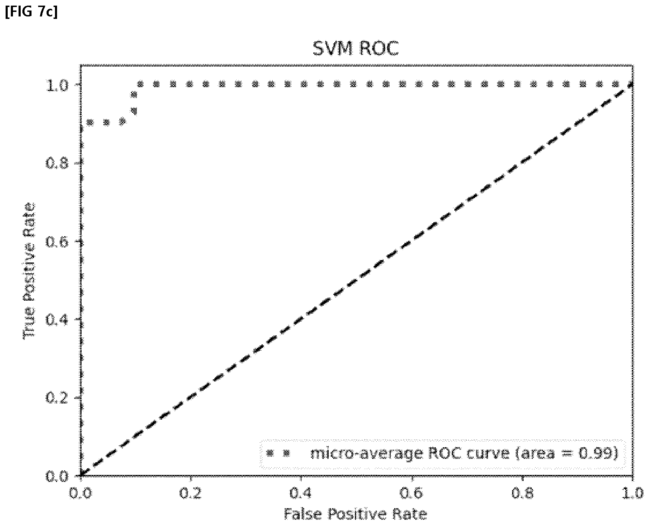

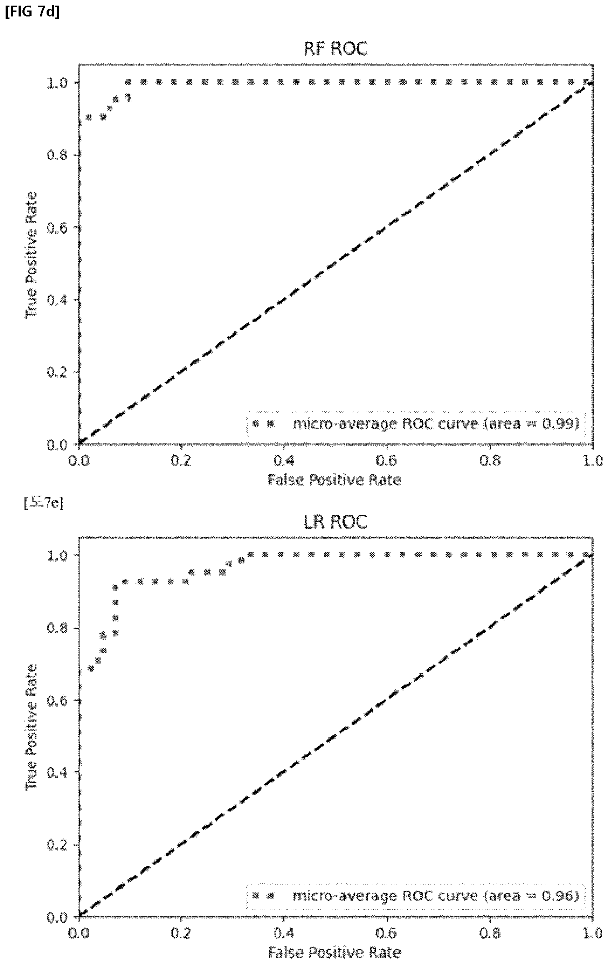

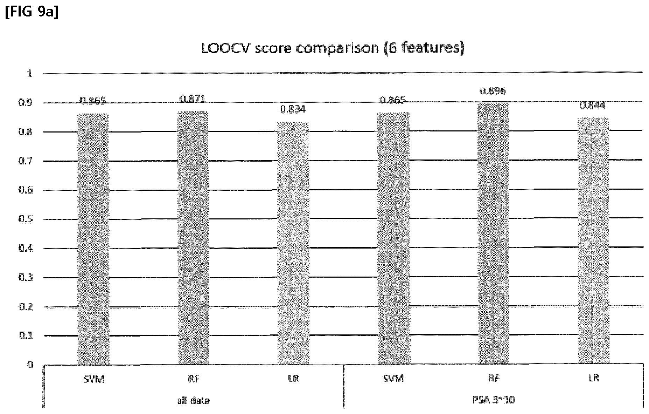

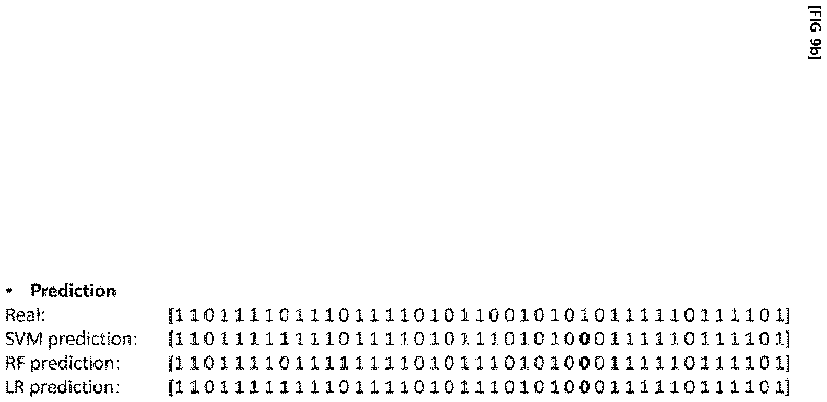

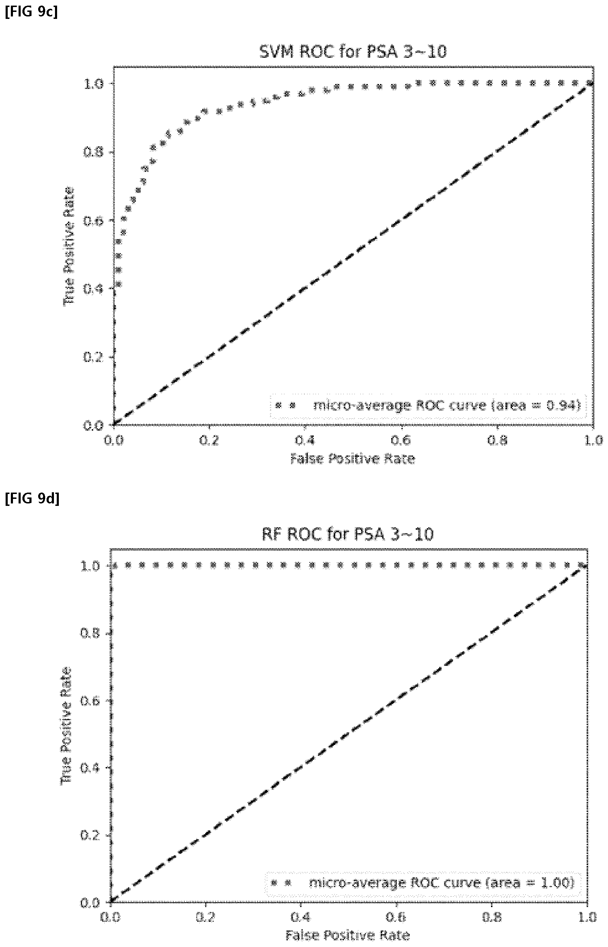

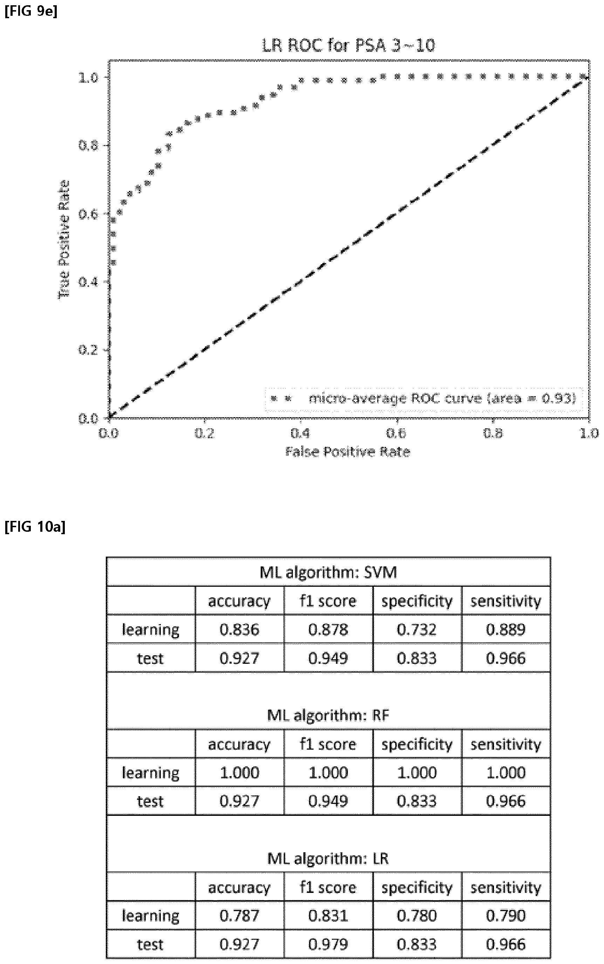

In one embodiment, when leave-one-out (LOO) and random forest (RF) were used as parameters, the marker combination is made to form a local maximum as a result of applying a recursive feature elimination with cross-validation (RFECV) algorithm. Preferably, the marker combination is performed using logistic regression (LR), and may be formed to have a leave-one-out cross-validation (LOOCV) value of 0.65 or more and an AUC value of 0.70 or more. As an example, such a combination may include a combination of miRNAs, such as hsa_miR_125b_5p, hsa_miR_141_5p, hsa_miR_17_3p, and hsa_miR_30c_5p, and the ROC curve of this combination is shown in FIG. 7. In another example, such a combination may include PCA3 as lncRNA, TMPRSS2:ERG as mRNA of a fusion gene, and the combination of hsa_miR_125b_5p, hsa_miR_141_5p, hsa_miR_17_3p, and hsa_miR_30c_5p as miRNAs. The ROC curve for the combination is shown in FIG. 9.

In one embodiment, the marker combination may be at least one selected from the group consisting of hsa_miR_375, hsa_miR_27b_3p, hsa_miR_31_5p, hsa_miR_125b_5p, hsa_miR_146a_3p, hsa_miR_146a_5p, hsa_miR_17_3p, hsa_miR_200b_3p, hsa_miR_21_5p, hsa_miR_1185_2_3p, hsa_miR_141_5p, hsa_miR_222_3p, hsa_miR_24_3p, hsa_miR_30a_3p, hsa_miR_30a_5p, hsa_miR_30b_5p, and hsa_miR_30c_5p, and specifically, hsa_miR_125b_5p, hsa_miR_141_5p, hsa_miR_17_3p, and hsa_miR_30c_5p.

In one embodiment, the marker combination may be (a) lncRNA and miRNA, (b) mRNA of a fusion gene and miRNA, or (c) lncRNA, miRNA, and mRNA of a fusion gene, wherein the lncRNA may be at least one selected from PCA3 and MALAT1, the mRNA of a fusion gene may be TMPRSS2:ERG, and the miRNA may be at least one selected from the group consisting of hsa_miR_125b_5p, hsa_miR_141_5p, hsa_miR_21_5p, hsa_miR_17_3p, hsa_miR_24_3p, hsa_miR_30a_5p, hsa_miR_222_3p, and hsa_miR_30c_5p.

In one embodiment, the marker combination may be a combination of lncRNA and mRNA of a fusion gene, the lncRNA may be PCA3 and MALAT1, and the mRNA of a fusion gene may be TMPRSS2:ERG.

In one embodiment, the marker is miRNA, and the miRNA is at least two selected from the group consisting of hsa_miR_125b_5p, hsa_miR_141_5p, hsa_miR_21_5p, hsa_miR_17_3p, hsa_miR_24_3p, hsa_miR_30a_5p, hsa_miR_222_3p, and hsa_miR_30c_5p.

In one embodiment, the marker combination is a combination of lncRNA, miRNA, and mRNA of a fusion gene, wherein the lncRNA is PCA3, the mRNA of a fusion gene is TMPRSS2:ERG, and the miRNA includes hsa_miR_125b_5p, hsa_miR_141_5p, hsa_miR_17_3p, and hsa_miR_30c_5p.

In one embodiment, primers that specifically bind to the lncRNA and/or the mRNA of a fusion gene, respectively, may consist of a base sequence as follows (SEQ ID NOs: 1 to 14).

| Primers of lncRNA or mRNA of fusion gene | Forward (5' → 3') | Reverse (5' → 3') |
|---|---|---|
| PCA3_4 | GAGAACAGGGGAGGGAGAG (SEQ ID NO: 1) | ACGTTCTGGGATACATGTGC (SEQ ID NO: 2) |
| PCA3_C2 | TGATAGGTGCAGCAAACCAC (SEQ ID NO: 3) | ACGTTCTGGGATACATGTGCAG (SEQ ID NO: 4) |
| TMPRSS2:ERG_R3 | CCTGGAGCGCGGCAGGAAGCCTTAT CAGTTG (SEQ ID NO: 5) | TCCTGCTGAGGGACGCGTGGGCTC ATCTTG (SEQ ID NO: 6) |

(continued)

| Primers of lncRNA or mRNA of fusion gene | Forward (5' → 3') | Reverse(5' → 3') |
|---|---|---|
| TMPRSS2:ERG_R6 | GGCAGGAACTCTCCTGAT(SEQ ID NO: 7) | CGTGGCACGATAACTCTG (SEQ ID NO: 8) |
| MALAT1_2 | GCAGGCGTTGTGCGTAGAG (SEQ ID NO: 9) | TTGCCGACCTCACGGATT (SEQ ID NO: 10) |
| MALAT1_4 | GGGTGTTTACGTAGACCAGAACC (SEQ ID NO: 11) | CTTCCAAAAGCCTTCTGCCTTAG (SEQ ID NO: 12) |
| MALAT1_6 | CTTCCCTAGGGGATTTCAGG (SEQ ID NO: 13) | GCCCACAGGAACAAGTCCTA (SEQ ID NO: 14) |
| β-actin | ATGTACCCTGGCATTGCCGAC (SEQ ID NO: 15) | GACTCGTCATACTCCTGCTTG (SEQ ID NO: 16) |
| 18s-rRNA | CCTGGATACCGCAGCTAGGA (SEQ ID NO: 17) | GCGGCGCAATACGAATGCCCC (SEQ ID NO: 18) |

In one embodiment, the miRNAs may consist of base sequences as follows.

| miRNA | Sequence(5' → 3') |
|---|---|
| hsa-miR-1185-2-3p | AUAUACAGGGGGAGACUCUCAU (SEQ ID NO: 19) |
| hsa-miR-125b-5p | UCCCUGAGACCCUAACUUGUGA (SEQ ID NO: 20) |
| hsa-miR-141-3p | UAACACUGUCUGGUAAAGAUGG (SEQ ID NO: 21) |
| hsa-miR-141-5p | CAUCUUCCAGUACAGUGUUGGA (SEQ ID NO: 22) |
| hsa-miR-146a-5p | UGAGAACUGAAUUCCAUGGGUU (SEQ ID NO: 23) |
| hsa-miR-17-3p | ACUGCAGUGAAGGCACUUGUAG (SEQ ID NO: 24) |
| hsa-miR-200b-3p | UAAUACUGCCUGGUAAUGAUGA (SEQ ID NO: 25) |
| h5a-miR-21-5p | UAGCUUAUCAGACUGAUGUUGA (SEQ ID NO: 26) |
| hsa-miR-222-3p | AGCUACAUCUGGCUACUGGGU (SEQ ID NO: 27) |
| hsa-miR-24-3p | UGGCUCAGUUCAGCAGGAACAG (SEQ ID NO: 28) |
| hsa-miR-27b-3p | UUCACAGUGGCUAAGUUCUGC (SEQ ID NO: 29) |
| hsa-miR-30a-3p | CUUUCAGUCGGAUGUUUGCAGC(SEQ ID NO: 30) |
| hsa-miR-30a-5p | UGUAAACAUCCUCGACUGGAAG (SEQ ID NO: 31) |
| hsa-miR-30b-Sp | UGUAAACAUCCUACACUCAGCU (SEQ ID NO: 32) |
| hsa-miR-30c-5p | UGUAAACAUCCUACACUCUCAGC (SEQ ID NO: 33) |
| hsa-miR-31-5p | AGGCAAGAUGCUGGCAUAGCU (SEQ ID NO: 34) |
| hsa-mir-375 | CCCCGCGACGAGCCCCUCGCACAAACCGGACCUGAGCGUUUUGUUCGUUCGGCUC GCGUGAGGC (SEQ ID NO: 35) |

The base sequences represented by SEQ ID NOs: 1 to 14 are the base sequences of primers specifically binding to lncRNA or mRNA of a fusion gene, the base sequences represented by SEQ ID NOs: 19 to 35 are the base sequences of miRNAs itself. Probes or primers specifically binding to miRNA may be designed with reference to the miRNA base sequence. The probes or primers may effectively detect lncRNA, miRNA, and/or mRNA of a fusion gene, which can be used as a diagnostic marker that can diagnose the onset or progression of prostate cancer, and the base sequence of a primer can be modified to a certain extent to detect lncRNA, miRNA, and/or mRNA of a fusion gene that can be used as a diagnostic marker. Those of ordinary skill in the art will easily understand that a base sequence that maintains 80% or more, preferably 90% or more, more preferably 95% or more, and most preferably 98% or more of homology by such artificial modification is a target prostate cancer diagnostic marker, and is equivalent to the base sequence of the present invention, which can significantly compare a difference in expression level between a normal subject and a subject with a prostate cancer disease.

A protein of each marker is known in the art, and for example, the sequence of each marker may be a human-derived sequence.

The term "biological sample" or "specimen" used herein includes any solid or liquid sample always obtained from the human body or a mammal, for example, a specific organ-derived tissue, cell, urine, saliva, semen, whole blood, plasma, or serum sample, but the present invention is not limited thereto.

In one embodiment, the biological sample for which the prostate diagnostic marker of the present invention is used may be at least one selected from the group consisting of prostate cancer tissue, cells, urine, saliva, semen, whole blood, plasma, and serum. Preferably, the biological sample for which the prostate cancer diagnostic marker of the present invention is used is urine.

In another embodiment, as the biological sample used in the present invention, for comparative analysis, a biological sample of a test subject requiring the diagnosis of prostate cancer as well as biological samples derived from a normal control and a specific type of prostate cancer control may be used.

The term "agent capable of measuring an expression level of lncRNA, miRNA, and/or mRNA of a fusion gene" refers to an agent that is used in a method of confirming whether corresponding lncRNA, miRNA, mRNA of a fusion gene and/or a fragment thereof included in a sample is expressed, and preferably, a primer, a probe, and/or an antibody that can specifically bind to a target gene used in a method such as reverse transcriptase polymerase chain reaction (RT-PCR), competitive RT-PCR, quantitative RT-PCR (qPCR), droplet digital PCR (ddPCR), sequencing, an RNase protection method, Northern blotting, and/or gene chip analysis. Preferably, the agent may be an agent that can measure the expression level of lncRNA, miRNA, and/or mRNA of a fusion gene.

In one embodiment, the present invention may detect a marker using RT-PCR and qPCR. RT-PCR refers to a technique of isolating RNA, such as lncRNA, miRNA, or mRNA, from a biological sample, synthesizing cDNA therefrom, and amplifying a specific gene in a large amount using a specific primer, or the combination of a primer and a probe. qPCR, also known as real-time PCR, is a PCR method to which a fluorescent material is applied, and a method of quickly and precisely analyzing the amplification of a target gene and its pattern by amplifying the target gene present in a sample, real-time detecting the emission level of a fluorescent material, and performing quantitative analysis during the reaction using a specific primer, or the combination of a primer and a probe, and is mainly used to screen high-speed large-quantity experimental results by confirming a change in expression of multiple target genes. This method is described in, for example, (Han, H.; Bearss, D. J.; Browne, L. W.; Calaluce, R.; Nagle, R. B.; Von Hoff, D. D., Identification of differentially expressed genes in pancreatic cancer cells using cDNA microarray. Cancer Res 2002, 62, (10), 2890-6. / Kwang Hyuck Lee, Quantification of DNA as a tumor marker in patients with pancreatic cancer, Journal of the Korean Society of Gastroenterology 2005, 46, 226-32).

The term "primer" used herein refers to a nucleic acid sequence having a short free 3' hydroxyl group, and a short nucleic acid sequence that can form a base pair with a complementary template and serve as a starting point for replicating a template strand. Primers may initiate DNA synthesis in the presence of a reagent (i.e., DNA polymerase or reverse transcriptase) for polymerization with a suitable buffer solution and an appropriate temperature, and four different nucleoside triphosphates.

The term "probe" used herein refers to a nucleic acid fragment such as RNA or DNA that is as short as a few bases or as long as hundreds of bases, can specifically bind to a gene or mRNA, and may be manufactured in the form of an oligonucleotide probe, a single-stranded DNA probe, a double-stranded DNA probe, or an RNA probe. The probe may be labeled to more easily detect the gene or mRNA.

The primer or probe of the present invention may be chemically synthesized using a phosphoramidite solid support method, or other widely-known methods. Such a nucleic acid sequence may also be modified using many means known in the art. Non-limiting examples of such modification include methylation, capping, substitution of natural nucleotides with one or more homologs, and modification between nucleotides, for example, modification into a uncharged linker (e.g., methyl phosphate, phosphotriester, phosphoroamidate, or carbamate) or a charged linker (e.g., phosphorothioate or phosphorodithioate).

In one embodiment of the present invention, expression levels of the biomarkers of the present invention were measured in urine samples of all prostate cancer patients. As a result, the expression levels of the biomarker, such as lncRNA, for PCA3 and MALAT1 and the biomarker, such as mRNA of a fusion gene, for TMPRSS2:ERG were shown to be remarkably up-expressed compared to a benign prostatic hyperplasia (BPH) control, so they were selected as markers. It was confirmed from urine samples of most of the prostate cancer patients that the miRNA biomarker was down-expressed compared to the BPH control, and all miRNA biomarkers used in the experiment were selected as markers.

In another embodiment of the present invention, the combination of lncRNA, miRNA, and/or mRNA of a fusion gene, which can be used as a biomarker for diagnosing prostate cancer, was deduced. The lncRNA, miRNA, and/or mRNA of a fusion gene were analyzed through qPCR in urine samples of 101 patients histologically diagnosed with prostate cancer and 62 persons of a non-tumor control, and a total of 20 genes, such as two lncRNAs (PCA3, MALAT1), one mRNA of a fusion gene (TMPRSS2:ERG), and 17 miRNAs (hsa_miR_375, hsa_miR_27b_3p, hsa_miR_31_5p, hsa_miR_125b_5p, hsa_miR_146a_3p, hsa_miR_146a_5p, hsa_miR_17_3p, hsa_miR_200b_3p, hsa_miR_21_5p, hsa_miR_1185_2_3p, hsa_miR_141_5p, hsa_miR_222_3p, hsa_miR_24_3p, hsa_miR_30a_3p, hsa_miR_30a_5p, hsa_miR_30b_5p, and hsa_miR_30c_5p) were selected. Most preferably, among these, a combination of four miRNAs (hsa_miR_125b_5p, hsa_miR_141_5p, hsa_miR_17_3p, and hsa_miR_30c_5p) in which a difference in expression level between the non-tumor control and the tumor group was observed, was selected, and one lncRNA (PCA3), one mRNA of a fusion gene (TMPRSS2:ERG), and the combination of four miRNAs (hsa_miR_125b_5p, hsa_miR_141_5p, hsa_miR_17_3p, and hsa_miR_30c_5p) were selected.

The usefulness of the combination of four miRNAs (hsa_miR_125b_5p, hsa_miR_141_5p, hsa_miR_17_3p, and hsa_miR_30c_5p) and one lncRNA (PCA3), and one mRNA of a fusion gene (TMPRSS2:ERG) and the combination of four miRNAs (hsa_miR_125b_5p, hsa_miR_141_5p, hsa_miR_17_3p, and hsa_miR_30c_5p), which had been selected as above, as detection markers was evaluated in a total of 163 urine samples acquired from prostate cancer patients (101 persons) and BPH patients (62 persons). As a result, it can be seen that prostate cancer can be effectively diagnosed using an agent for measuring the expression level of the combination of four miRNAs (hsa_miR_125b_5p, hsa_miR_141_5p, hsa_miR_17_3p, and hsa_miR_30c_5p), or an agent for measuring the expression levels of six markers, such as lncRNA (PCA3), mRNA of a fusion gene (TMPRSS2:ERG), and/or miRNAs (hsa_miR_125b_5p, hsa_miR_141_5p, hsa_miR_17_3p, and hsa_miR_30c_5p).

In addition, the present invention relates to a method of providing information for diagnosing prostate cancer using a composition or kit for diagnosing prostate cancer.

Specifically, the method of providing information for diagnosing prostate cancer according to the present invention includes measuring an expression level of miRNA in a biological sample isolated from a subject; and comparing the expression level of miRNA with an expression level of miRNA of a normal control sample.

After measuring the expression level of miRNA in the measuring of an expression level, in the comparing of the expression levels, the expression level of miRNA may be compared with that of the corresponding miRNA in the normal control sample to determine whether prostate cancer has developed or progressed when the expression level of the marker significantly increases or decreases.

In one embodiment, the expression level of miRNA may be measured by RT-PCR, competitive RT-PCR, quantitative RT-PCR (qPCR), droplet digital PCR (ddPCR), sequencing, an RNase protection method, Northern blotting, and/or gene chip analysis, but the present invention is not limited thereto.

In one embodiment, the measuring of an expression level may include: extracting miRNAs including hsa_miR_375, hsa_miR_27b_3p, hsa_miR_31_5p, hsa_miR_125b_5p, hsa_miR_146a_3p, hsa_miR_146a_5p, hsa_miR_17_3p, hsa_miR_200b_3p, hsa_miR_21_5p, hsa_miR_1185_2_3p, hsa_miR_141_5p, hsa_miR_222_3p, hsa_miR_24_3p, hsa_miR_30a_3p, hsa_miR_30a_5p, hsa_miR_30b_5p and/or hsa_miR_30c_5p from a biological sample isolated from a subject; synthesizing cDNA from the extracted miRNA; amplifying the cDNA using a primer pair specific for the synthesized cDNA; and detecting the amplified cDNA.

In one embodiment, the biological sample may be tissue, cells, urine, saliva, semen, whole blood, plasma, and/or serum, acquired from a subject. Preferably, the biological sample is urine. Preferably, the urine is collected before/after a subject's prostate massage or digital rectal examination (DRE).

In one embodiment, the comparing of the expression levels may be performed using a conventional statistical analysis method. Preferably, the algorithm is performed by logistic regression (LR). When the expression levels of multiple biomarkers are analyzed using an LR algorithm, prostate cancer may be diagnosed and prediction accuracy may be improved. The algorithm will be described below.

In one embodiment, the comparing of the expression levels may be performed using a machine learning algorithm. Preferably, the algorithm may be selected from the group consisting of LR, a support vector machine (SVM), a random forest, and a multi-layer perceptron. When the expression levels of the multiple biomarkers are analyzed using the machine leaning algorithm, the diagnosis and accuracy of predicting prostate cancer may be further improved. The algorithm will be described below.

The comparing of the expression levels of miRNA may be performed using LR, a leave-one-out cross-validation (LOOCV) value may be 0.65 or more, and an area under the curve (AUC) value may be 0.70 or more.

In one embodiment, the method of providing information for diagnosing prostate cancer according to the present invention may further use non-protein clinical information of a patient, that is, clinical information other than markers, in addition to the result of analyzing the biomarkers. The non-protein clinical information includes, for example, a patient's age, gender, body weight, eating habits, and body mass, ultrasound examination, computed tomography (CT), magnetic resonance imaging (MRI), angiography, endoscopic retrograde pancreatography, endoscopic ultrasound, tumor markers, and/or laparoscopy.

In one embodiment, the measuring of an expression level may further include measuring an expression level of lncRNA, mRNA of a fusion gene, or a combination thereof. Here, the lncRNA may be at least one selected from PCA3 and MALAT1, and the mRNA of a fusion gene may be TMPRSS2:ERG. In addition, the comparing of the expression level may further include comparing the expression level of the lncRNA, mRNA of a fusion gene, or combination thereof with that of lncRNA, mRNA of a fusion gene, or a combination thereof in a normal control sample. After the expression level of lncRNA, mRNA of a fusion gene and/or a fragment thereof may be measured in the measuring of an expression level, in the comparing of the expression levels, the expression level of lncRNA, mRNA of a fusion gene and/or a fragment thereof may be compared with that of corresponding lncRNA, mRNA of a fusion gene and/or a fragment thereof in the normal control sample to determine whether prostate cancer has developed or progressed when the expression level of a marker significantly increases or decreases.

In one embodiment, the expression level of miRNA, lncRNA, mRNA of a fusion gene, or a combination thereof may be measured by RT-PCR, competitive RT-PCR, qPCR, ddPCR, sequencing, a RNase protection method, Northern blotting, and/or gene chip analysis, but the present invention is not limited thereto.

In one embodiment, the measuring of an expression level may include: extracting the miRNA, lncRNAs including PCA3 and MALAT1, mRNA of a fusion gene including TMPRSS2:ERG, or a combination thereof from a biological sample isolated from a subject; synthesizing cDNA from the extracted miRNA, lncRNAs and mRNA of a fusion gene, or combination thereof; amplifying the cDNA using a primer set specific for the synthesized cDNA; and detecting the amplified cDNA.

In one embodiment, the biological sample may be tissue, cells, urine, saliva, semen, whole blood, plasma, and/or serum, acquired from a subject. Preferably, the biological sample is urine. Preferably, the urine is collected before/after a subject's prostate massage or digital rectal examination (DRE).

In one embodiment, the comparing of the expression levels may be performed using a conventional statistical analysis method. Preferably, the algorithm may be performed by LR. When the expression levels of multiple biomarkers are analyzed using an LR algorithm, prostate cancer may be diagnosed and prediction accuracy may be improved. The algorithm will be described below.

In one embodiment, the comparing of the expression levels may be performed using a machine learning algorithm. Preferably, the algorithm may be selected from the group consisting of LR, a support vector machine (SVM), a random forest, and a multi-layer perceptron. When the expression levels of the multiple biomarkers are analyzed using the machine leaning algorithm, the diagnosis and accuracy of predicting prostate cancer may be further improved. The algorithm will be described below.

In one embodiment, the method of providing information for diagnosing prostate cancer according to the present invention may further use non-protein clinical information of a patient, that is, clinical information other than markers, in addition to the result of analyzing the biomarkers. The non-protein clinical information includes, for example, a patient's age, gender, body weight, eating habits, and body mass, ultrasound examination, computed tomography (CT), magnetic resonance imaging (MRI), angiography, endoscopic retrograde pancreatography, endoscopic ultrasound, tumor markers, and/or laparoscopy.

The comparing of the expression level of miRNA with that of lncRNA, mRNA of a fusion gene, or combination thereof may be performed by LR, a leave-one-out cross-validation (LOOCV) value may be 0.65 or more, and an area under the curve (AUC) value may be 0.70 or more.

In addition, the present invent ion relates to a kit for diagnosing prostate cancer, including the composition for diagnosing prostate cancer.

In one embodiment, the kit for diagnosing prostate cancer of the present invention may be used to diagnose prostate cancer in a subject by measuring an expression level of a prostate cancer diagnostic marker, such as lncRNA, miRNA, and/or mRNA of a fusion gene. The miRNA combination is the same as above, and two or more of the biomarkers such as lncRNA, miRNA, and/or mRNA of a fusion gene may be used in combination, and the combination of these biomarkers is the same as above. Preferably, the combination may include one or more of one lncRNA (PCA3) and one mRNA of a fusion gene (TMPRSS2:ERG), and one or more of four miRNAs (hsa_miR_125b_5p, hsa_miR_141_5p, hsa_miR_17_3p, and hsa_miR_30c_5p). Accordingly, the kit for diagnosing prostate cancer of the present invention may include a composition, solution, or device with one or more components, which are suitable for the analysis method, in addition to a polynucleotide, primer, probe, or antibody for identifying one or more of one lncRNA (PCA3) and one mRNA of a fusion gene (TMPRSS2:ERG), and one or more of four miRNAs (hsa_miR_125b_5p, hsa_miR_141_5p, hsa_miR_17_3p, and hsa_miR_30c_5p).

In one embodiment, the kit for diagnosing prostate cancer according to the present invention may include essential elements for RT-PCR. The RT-PCR kit may include test tubes or appropriate different containers, buffer solutions (the pH and magnesium concentration vary), deoxynucleotides (dNTPs), enzymes such as Taq-polymerase and reverse transcriptase, DNase, an RNAse inhibitor, DEPC-water, and sterile water, other than primer pairs specific for the gene. In addition, primer pairs specific for a gene used as a quantitative control may be included.

In another embodiment, the kit for diagnosing prostate cancer according to the present invention may include essential elements for gene chip analysis. The kit for gene chip analysis may include a substrate to which cDNA corresponding to a gene or its fragment is attached as a probe, and a reagent, agent, and enzyme for manufacturing a fluorescence-labeled probe. In addition, the substrate may include cDNA corresponding to a quantitative control gene or its fragment.

In another embodiment, the kit for diagnosing prostate cancer of the present invention may further include an agent for measuring a gene expression level of a prostate cancer diagnostic biomarker known in the art, other than the biomarkers (e.g., lncRNA (PCA3), mRNA of a fusion gene (TMPRSS2:ERG), and miRNA (hsa_miR_125b_5p, hsa_miR_141_5p, hsa_miR_17_3p and hsa_miR_30c_5p)).

A material that specifically recognizes the biomarker of the present invention may be separately dispensed in a compartmentalized container, and thus, the present invention provides a compartmentalized device and/or tool, which contains molecules that can specifically recognize the marker of the present invention.

In one embodiment, the kit for diagnosing prostate cancer of the present invention may further include reagents, devices, and/or information processing means with a built-in algorithm for detecting one or more biomarkers of the present invention, and through the algorithm, the detection result for biomarkers may be correlated with the diagnosis of prostate cancer. The correlation involves training the algorithm to deduce a pattern for the difference in expression level by comparing the expression levels of one or more markers in a prostate cancer screening candidate, a normal control, or a patient with a certain type of prostate cancer.

In one embodiment, the training of the algorithm includes constructing an algorithm mapping the expression level of a biomarker given as an input value with a diagnostic result given as an output value; executing the constructed algorithm to map the marker expression level and the diagnosis or absence of prostate cancer; and executing the algorithm while changing the input value of the constructed algorithm and the output value obtained therefrom to realize an optimal algorithm mapping architecture. The optimal algorithm mapping identifies a significant difference using the marker expression level in the normal control or patient with specific prostate cancer and the marker expression level in the prostate cancer screening candidate, which is used for the diagnosis or absence of prostate cancer.

In the present invention, a known algorithm may be used, and may be selected from the group consisting of LR, SVM, random forest, and multi-layer-perceptron, but the present invention is not limited thereto.

In one embodiment, the biological sample used in the kit for diagnosing prostate cancer according to the present invention may be prostate cancer tissue, cells, urine, saliva, semen, whole blood, plasma, and/or serum, and preferably, urine, and for comparative analysis, samples derived from a patient requiring the determination of prostate cancer, and samples derived from a normal control or a prostate cancer control.

In addition, the present invention provides a method of diagnosing prostate cancer using the composition or kit for diagnosing prostate cancer.

Hereinafter, the present invention will be described in further detail with reference to examples. These examples are intended to illustrate the present invention in further detail, and the scope of the present invention is not limited to these examples.

**<Examples>**

**1. Materials and methods**

**Preparation of urine samples**

In this experiment, urine samples from 62 patients with BPH and urine samples from 101 prostate cancer patients, provided by Asan Medical Center in Seoul, were used. In prostate cancer and benign BPH, self-urinated urine before the start of treatment and urine urinated immediately after digital rectal examination, and in a surgery patient, urine collected during catheter insertion on the day of surgery (collecting a urine sample so that a urine volume was 30 mL or more) were collected and then stored in a -80 °C ultra-low temperature freezer.

**Extraction of lncRNA, miRNA, and mRNA of fusion gene from urine samples**

To extract lncRNA, miRNA, and mRNA of a fusion gene from a urine sample, a QIAGEN product (cat no.

55114) was used. 4 mL of the urine sample was added to a 50 mL centrifuge tube into which 500 µL of proteinase K was pipetted, and 4 mL of buffer ACL (including carrier RNA as needed) and 1.0 mL of buffer ATL were added and then mixed by pulse-vertexing for 30 seconds. Afterward, the resulting product was incubated at 60 °C for 30 minutes, and 9.0 mL of buffer ACB was added to a lysate in the tube and completely mixed by pulse-vortexing for 15 to 30 seconds. Subsequently, the lysate-buffer ACB mixture was incubated in the tube on ice for 5 minutes, a QIAamp Mini column was inserted into a VacConnector (QIAvac 24 Plus), and a 20-mL tube extender was inserted into the open QIAamp Mini column. The lysate-buffer ACB mixture was gently applied to the tube extender of the QIAamp Mini column, and after a vacuum pump was turned on to completely suck all of the lysate through the column, the vacuum pump was turned off. 600 µL of buffer ACW1 was applied to the QIAamp Mini column, and after turning on the vacuum pump while opening a column lid, all of the buffer ACW1 was extracted through a QIAamp mini column and then the vacuum pump was turned off. 750 µL of buffer ACW2 was applied to the QIAamp Mini column, and after the vacuum pump was turned on while opening the column lid, all of the buffer ACW2 was extracted through the QIAamp Mini column and then the vacuum pump was turned off. 750 µL of ethanol (96-100%) was applied to the QIAamp Mini column, and after the vacuum pump was turned on while opening the column lid, all of the ethanol was extracted through a spin column and then the vacuum pump was turned off. The QIAamp Mini column was put into a 2-mL clean collection tube, and centrifuged at the maximum speed (20,000 × g, 14,000 rpm) for 3 minutes. The QIAamp Mini column was put into a clean 2-mL collection tube, the lid was opened and then the assembly was incubated at 56 °C for 10 minutes to completely dry a membrane. The QIAamp Mini column was put into a clean 1.5-mL elution tube, the 2 mL collection tube was discarded, 20 to 150 µL of buffer AVE was gently applied to the center of the QIAamp Mini membrane, and the lid was closed, followed by incubating at room temperature for 3 minutes. Finally, the resulting product was centrifuged in a microcentrifuge at the maximum speed (20,000 × g, 14,000 rpm) for 1 minute to elute a nucleic acid.

To extract miRNA from a urine sample, a QIAGEN product (cat no. 55114) was used. 3 mL of the urine sample was added to a 50-mL centrifuge tube into which 400 µL of proteinase K was pipetted, and 3.2 mL of buffer ACL (without carrier RNA) and 1.0 mL of buffer ATL were added and mixed by pulse-vortexing for 30 seconds. Afterward, the resulting product was incubated at 60 °C for 30 minutes, and 9.0 mL of buffer ACB and 7.0 mL of isopropanol were added to the lysate in the tube and completely mixed by pulse-vortexing for 15 to 30 seconds. Afterward, the lysate-buffer ACB mixture was incubated in the tube on ice for 5 minutes, the QIAamp Mini column was inserted into a VacConnector (QIAvac 24 Plus), and a 20-mL tube extender was inserted into an open QIAamp Mini column. The lysate-buffer ACB mixture was gently applied to the tube extender of the QIAamp Mini column, and after a vacuum pump was turned on to complexly suck all of the lysate through the column, the vacuum pump was turned off. 600 µL of buffer ACW1 was applied to the QIAamp Mini column, the vacuum pump was turned on while opening a column lid, all of the buffer ACW1 was extracted from the QIAamp Mini column, and then the vacuum pump was turned off. 750 µL of buffer ACW2 was applied to the QIAamp Mini column, the vacuum pump was turned on while opening the column lid, all of the buffer ACW2 was extracted from the QIAamp Mini column, and then the vacuum pump was turned off. 750 µL of ethanol (96 to 100%) was applied to the QIAamp Mini column, and after the vacuum pump was turned on while opening the column lid, all of the ethanol was extracted from a spin column and then the vacuum pump was turned off. The QIAamp Mini column was put into a 2-mL clean collection tube, and centrifuged at the maximum speed (20,000 × g, 14,000 rpm) for 3 minutes. The QIAamp Mini column was put into a clean 2-mL collection tube, the lid was opened and then the assembly was incubated at 56 °C for 10 minutes to completely dry a membrane. The QIAamp Mini column was put into a clean 1.5-mL elution tube, the 2-mL collection tube was discarded, 20 to 150 µL of buffer AVE was gently applied on the center of the QIAamp Mini membrane, and the lid was closed, followed by incubating at room temperature for 3 minutes. Finally, centrifugation was performed in a microcentrifuge at the maximum speed (20,000 × g, 14,000 rpm) for 1 minute, thereby eluting a nucleic acid.

**Synthesis of cDNAs of lncRNA, miRNA, and mRNA of fusion gene extracted from urine** samples

To synthesize cDNAs from, miRNA, and mRNA of a fusion gene extracted from a urine sample, a QIAGEN product (cat no. 235311) was used. Each template RNA was thawed on ice, gDNA Wipeout Buffer, Quantiscript Reverse Transcriptase, Quantiscript RT Buffer, RT Primer Mix, and RNase-free water were thawed at room temperature (15 to 25 °C). According to Table 1 below, components for a genomic DNA removal reaction were prepared on ice, and after mixing, stored on ice. Afterward, after incubating the mixture at 42 °C for 2 minutes and immediately placing on ice, a reverse transcription master mix was prepared on ice according to Table 2 below. The template RNA (14 µL) was added to each tube containing the reverse transcription master mix and incubated at 42 °C for 15 minutes and then at 95 °C for 3 minutes, thereby inactivating the Quantiscript reverse transcriptase. An aliquot of each finished reverse transcription reaction was added to a real-time PCR mix.

[Table 1] Components for genomic DNA removal reaction

| Component | Volume/reaction | Final concentration |
|---|---|---|
| gDNA Wipeout Buffer, 7x | 2 µl | 1x |
| Template RNA | Variable (up to 1 µg*) | |
| RNase-free water | Variable | |
| Total volume | 14 µl | — |

[Table 2] Components for reverse transcription reaction

| Component | Volume/reaction | Final concentration |
|---|---|---|
| Reverse-transcription master mix | | |
| Quantiscript Reverse Transcriptase* | 1 µl | |
| Quantiscript RT Buffer, 5x† | 4 µl | 1x |
| RT Primer Mix‡ | 1 µl | |
| Template RNA | | |
| Entire genomic DNA elimination reaction (step 3) | 14 µl (add at step 5) | |
| Total volume | 20 µl | — |

To synthesize cDNA from miRNA extracted from a urine sample, a QIAGEN product (cat no. 339340) was used. Each template RNA sample was diluted to 5 ng/µL using nuclease-free water, components for a reverse transcription reaction were prepared on ice according to Tabel 3 below and mixed, followed by storing on ice. By incubating the mixture at 42 °C for 60 minutes and then at 95 °C for 5 minutes, the reverse transcriptase was heat-inactivated and then immediately cooled to 4 °C. Afterward, an miRNA PCR assay was performed.

[Table 3] Setup for reverse transcription reaction per sample

| Component | miRNA PCR Assay | miRNome PCR Panels : Human, Mouse & Rat (Panel I) | miRNome PCR Panels : Human, Mouse & Rat (Panel I+II) | Focus PCR Panel: Cancer (1 x 96 assays) | Custom PCR Panel: ≤192 miRNAs analyzed per sample | Custom PCR Panel: 193–384 miRNAs analyzed per sample |
|---|---|---|---|---|---|---|
| 5x miRCURY SYBR® Green RT Reaction Buffer | 2 µl | 4 µl | 8 µl | 2 µl | 2 µl | 4 µl |
| RNase-free water | 4.5 µl | 9 µl | 18 µl | 4.5 µl | 4.5 µl | 9 µl |
| 10x miRCURY RT Enzyme Mix | 1 µl | 2 µl | 4 µl | 1 µl | 1 µl | 2 µl |
| UniSp6 RNA spike-in (optional) | 0.5 µl | 1 µl | 2 µl | 0.5 µl | 0.5 µl | 1 µl |
| Template RNA (5 ng/µl) | 2 µl | 4 µl | 8 µl | 2 µl | 2 µl | 4 µl |
| Total reaction volume | 10 µl* | 20 µl* | 40 µl* | 10 µl* | 10 µl* | 20 µl* |

* All volumes refer to corresponding PCR reaction volumes of 10 µl. For the Rotor-Disc 100, a reaction volume of 20 µl is recommended, double the amount of all reagents in the Reverse Transcription setup.

**Quantitative PCR (qPCR)**

TO measure the expression of each marker, qPCR was performed three times using Stratagene Mx3000P (Agilent Technologies, California, USA). To quantify lncRNA, miRNA, and mRNA of a fusion gene, the cDNAs that had been synthesized in the previous step were used as PCR templates, and for amplification and detection, ORA™qPCR Green ROX L Mix, 2X (highQu, Kraichtal, Germany) was used. The final volume of each tube was 10 µL consisting of ORA™qPCR Green ROX L Mix, 2X (5.0 µL), pre-diluted cDNA (4.0 µL), and a pre-mixed primer set (1 µL). To measure the expression levels of lncRNA and mRNA of a fusion gene, the concentrations of cDNA, and primers for lncRNA and mRNA of a fusion gene were 160 to 200 ng, and 500 nM at the final volume. qPCR cycling conditions were as follows: After initial denaturation at 95 °C for 15 minutes, 50 cycles of template denaturation at 95 °C for 15 seconds and elongation at 60 °C for 60 seconds were performed, and one cycle of melting curve analysis was finally performed at 60 to 95 °C. To measure an miRNA expression level, a cDNA concentration was 12 to 16 ng at the final volume. An miRCURY LNA™ miRNA PCR assay (QIAGEN, Hilden, Germany) was used as a primer for miRNA detection, and before use, was pre-diluted with 220 µL of nuclease-free water. qPCR cycling conditions were as follows: after initial denaturation at 95 °C for 2 minutes, 50 cycles of template denaturation at 95 °C for 10 seconds, elongation at 56 °C for 60 seconds were performed, and one cycle of melting curve analysis at 56 to 95 °C was finally performed. Raw data measured by qPCR was analyzed using MxPro software. All primer sets are listed below.

[Table 4] Primer sequences (lncRNA or mRNA of a fusion gene)

| Primers of lncRNA or mRNA of fusion gene | Forward(5' → 3') | Reverse(5' → 3') |
|---|---|---|
| PCA3_4 | GAGAACAGGGGAGGGAGAG (SEQ ID NO: 1) | ACGTTCTGGGATACATGTGC (SEQ ID NO: 2) |
| PCA3_C2 | TGATAGGTGCAGCAAACCAC (SEQ ID NO: 3) | ACGTTCTGGGATACATGTGCAG (SEQ ID NO: 4) |

(continued)

| Primers of lncRNA or mRNA of fusion gene | Forward(5' → 3') | Reverse(5' → 3') |
|---|---|---|
| TMPRSS2:ERG_R3 | CCTGGAGCGCGGCAGGAAGCCTTAT CAGTTG (SEQ ID NO: 5) | TCCTGCTGAGGGACGCGTGGGCTC ATCTTG (SEQ ID NO: 6) |
| TMPRSS2:ERG_R6 | GGCAGGAACTCTCCTGAT (SEQ ID NO: 7) | CGTGGCACGATAACTCTG (SEQ ID NO: 8) |
| MALAT1_2 | GCAGGCGTTGTGCGTAGAG (SEQ ID NO: 9) | TTGCCGACCTCACGGATT (SEQ ID NO: 10) |
| MALAT1_4 | GGGTGTTTACGTAGACCAGAACC (SEQ ID NO: 11) | CTTCCAAAAGCCTTCTGCCTTAG (SEQ ID NO: 12) |
| MALAT1_6 | CTTCCCTAGGGGATTTCAGG (SEQ ID NO: 13) | GCCCACAGGAACAAGTCCTA (SEQ ID NO: 14) |
| β-actin | ATGTACCCTGGCATTGCCGAC (SEQ ID NO: 15) | GACTCGTCATACTCCTGCTTG (SEQ ID NO: 16) |
| 18s-rRNA | CCTGGATACCGCAGCTAGGA (SEQ ID NO: 17) | GCGGCGCAATACGAATGCCCC (SEQ ID NO: 18) |

[Table 5] Sequences (miRNA) from miRBase or GeneGlobe of QIAGEN

| miRNA | Sequence(5' → 3') |
|---|---|
| hsa-miR-1185-2-3p | AUAUACAGGGGGAGACUCUCAU (SEQ ID NO: 19) |
| hsa-miR-125b-5p | UCCCUGAGACCCUAACUUGUGA (SEQ ID NO: 20) |
| hsa-miR-141-3p | UAACACUGUCUGGUAAAGAUGG (SEQ ID NO: 21) |
| hsa-miR-141-5p | CAUCUUCCAGUACAGUGUUGGA (SEQ ID NO: 22) |
| hsa-miR-146a-5p | UGAGAACUGAAUUCCAUGGGUU (SEQ ID NO: 23) |
| hsa-miR-17-3p | ACUGCAGUGAAGGCACUUGUAG (SEQ ID NO: 24) |
| hsa-miR-200b-3p | UAAUACUGCCUGGUAAUGAUGA (SEQ ID NO: 25) |
| hsa-miR-21-5p | UAGCUUAUCAGACUGAUGUUGA (SEQ ID NO: 26) |
| hsa-miR-222-3p | AGCUACAUCUGGCUACUGGGU (SEQ ID NO: 27) |
| hsa-miR-24-3p | UGGCUCAGUUCAGCAGGAACAG (SEQ ID NO: 28) |
| hsa-miR-27b-3p | UUCACAGUGGCUAAGUUCUGC (SEQ ID NO: 29) |
| hsa-miR-30a-3p | CUUUCAGUCGGAUGUUUGCAGC (SEQ ID NO: 30) |
| hsa-miR-30a-5p | UGUAAACAUCCUCGACUGGAAG (SEQ ID NO: 31) |
| hsa-miR-30b-5p | UGUAAACAUCCUACACUCAGCU (SEQ ID NO: 32) |
| hsa-miR-30c-5p | UGUAAACAUCCUACACUCUCAGC (SEQ ID NO: 33) |
| hsa-miR-31-5p | AGGCAAGAUGCUGGCAUAGCU (SEQ ID NO: 34) |

(continued)

| miRNA | Sequence(5' → 3') |
|---|---|
| hsa-mir-375 | CCCCGCGACGAGCCCCUCGCACAAACCGGACCUGAGCGUUUUGUUCGUUCGGCUC GCGUGAGGC (SEQ ID NO: 35) |

**Single biomarker analysis**

To investigate the diagnostic ability of lncRNA, miRNA and mRNA of a fusion gene biomarkers, the expression levels of 17 miRNAs, 2 lncRNAs, and one mRNA of a fusion gene in 163 urine cDNA samples (62 BPH samples and 101 PCa samples) were measured by qPCR. The expression profiles of all biomarkers were normalized with 18s-rRNA and β-actin. The gene expression trend between BPH and PCa is shown in FIG. 2. The lncRNA biomarkers for PCA3 and MALAT1 and the mRNA biomarker of a fusion gene for TMPRSS2:ERG show up-regulation in PCa patients. All miRNA biomarkers for 17 miRNAs were detected to be down-regulated in PCa. Specifically, the diagnostic ability of each biomarker was evaluated with an ROC curve. lncRNA and miRNA showed the highest AUC values, 0.79 and 0.85, respectively (FIG. 3 and Tables 6 to 8).

[Table 6] AUC values (lncRNA/mRNA of a fusion gene/miRNA)

| Primer | AUC | Primer | AUC | Primer | AUC |
|---|---|---|---|---|---|
| MALAT1_2 | 0.68 | hsa-miR-1185-2-3p | 0.6 | hsa-miR-24-3p | 0.87 |
| MALAT1_4 | 0.79 | hsa-miR-125b-5p | 0.87 | hsa-miR-27b-3p | 0.89 |
| MALAT1_6 | 0.77 | hsa-miR-141-3p | 0.74 | hsa-miR-30a-3p | 0.73 |
| PCA3_4 | 0.76 | hsa-miR-141-5p | 0.86 | hsa-miR-30a-5p | 0.88 |
| PCA3_C2 | 0.76 | hsa-miR-146a-5p | 0.76 | hsa-miR-30b-5p | 0.80 |
| TMPRSS2:ER G_R3 | 0.77 | hsa-miR-17-3p | 0.87 | hsa-miR-30c-5p | 0.86 |
| TMPRSS2:ER G_R6 | 0.75 | hsa-miR-200b-3p | 0.82 | hsa-miR-31-5p | 0.75 |
| | | hsa-miR-21-5p | 0.85 | hsa-mir-375 | 0.76 |
| | | hsa-miR-222-3p | 0.90 | | |

[Table 7] Trend of lncRNA and mRNA of fusion gene biomarkers

| Primer | Reference | Experiment |
|---|---|---|
| MALAT1_2[1,2] | UP | UP |
| MALAT1_4[1,2] | UP | UP |
| MALAT1_6[1,2] | UP | UP |
| PCA3_4[3,4] | UP | UP |
| PCA3_C2[3,4] | UP | UP |
| TMPRSS2:ERG_R3[5] | UP | UP |
| TMPRSS2:ERG_R6[5] | UP | UP |
| UP= up-regulated in cancer / Down= down-regulated in cancer (1. Xue, D et al. Journal of Cellular and Molecular Medicine, 22(6), 3223-3237. (2018); 2. Wang, F., et al. Oncotarget, 5(22), 11091-11102. (2014); 3. Bussemakers, et al. Cancer Research, 59(23), 5975-5979. (1999); 4. Mao, Zujie et al, Medicine vol. 97,42 (2018); 5. Koo, Kevin M., et al., Scientific reports 6.1: 1-6 (2016); 6. Urbinati, Giorgia, et al. Molecular Therapy-Nucleic Acids 5 (2016)) | | |

[Table 8] Trend of miRNA biomarker

| Primer | Reference | Experiment | Primer | Reference | Experiment |
|---|---|---|---|---|---|
| hsa-miR-1185-2-3p | Up[2] | Down | hsa-miR-222-3p | Down[1,4] | Down |
| hsa-miR-125b-5p | Up or DOWN[1,4] | Down | hsa-miR-24-3p | Down[1] | Down |
| hsa-miR-141-3p | Dowri(hsa-mir-141)[1,4] | Down | hsa-mir-375 | Up or Down [1] | Down |
| hsa-miR-141-5p | Down(hsa-mir-141)[1,4] | Down | hsa-miR-27b-3p | Down[1,4] | Down |
| hsa-miR-146a-5p | Down[1] | Down | hsa-miR-30a-3p | Up[1] | Down |
| hsa-miR-31-5p | Down[1] | Down | hsa-miR-30a-5p | Up or Down[1,4] | Down |
| hsa-miR-17-3p | Up(hsa-mir-17) or Down[1,2,5] | Down | hsa-miR-30b-5p | Up or Down[1,4] | Down |
| hsa-miR-200b-3p | Down [1] | Down | hsa-miR-30c-5p | Up or Down[1,4] | Down |
| hsa-miR-21-5p | Down(hsa-mir-21)[1] | Down | | | |

*mir = precursor sequence / miR = mature sequence
*Up= up-regulated in cancer / Down= down-regulated in cancer
(1. FredsøJ. et al. Eur. Urol. Focus 4, 825-833 (2018); 2. Urabe, F. et al. Clin. Cancer Res. 25, 3016-3025 (2019); 3. Xueping Zhang et al. Clin Exp Metastasis 26(8):965-79(2009); 4. Kati P Porkka et al. Cancer Res 67(13):6130-5 (2007))

**Conventional statistical analysis method and data preprocessing before machine learning**

Based on the previous single biomarker analysis, normalized expressions ($-\Delta Ct$) of biomarkers for lncRNA, miRNA, and mRNA of a fusion gene were combined. Before combining normalized expressions ($-\Delta Ct$), data pre-processing was performed: First, among the biomarkers targeting the same lncRNA or mRNA of a fusion gene, the biomarker primers (PCA3_4, TMPRSS2:ERG_R3 and MALAT1_6) for lncRNA or mRNA of a fusion gene were selected by comparing AUCs. However, in the case of the TMPRSS2:ERG fusion gene, from the biomarker (TMPRSS2:ERG Fusion_R6) showing the highest AUC, No CT was frequently observed (50% or more), so the second best (TMPRSS2:ERG Fusion_R3, No Ct ratio 5%) was selected. Second, to prevent data imbalance, blank-normalized expression in the same biomarker was processed as the minimum normalized expression. Before applying the satisfied data set to machine learning, min-max scaling was performed.

**Selection of biomarker**

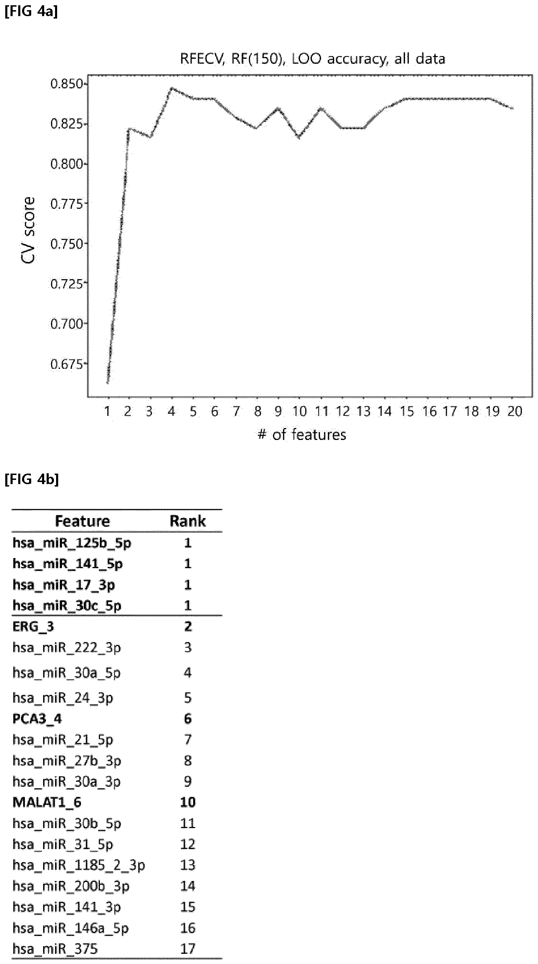

To improve the performance of PCa/BPH classifiers and provide robustness to the potential perturbation of a single biomarker, multi-biomarker-based PCa/BPH classifiers were targeted through various machine learning algorithms. For more excellent performance, to find the optimal biomarker combination, the recursive feature elimination with cross-validation (RFECV) algorithm was applied. As a RFECV parameter, leave-one-out (LOO) and random forest (RF) were used. LOO is suitable for minimizing group bias, and RF may provide feature importance in an additional analysis. By removing features with low feature importance through RFECV, the model performance may be calculated according to the number of features. That is, as a result of RFECV, the best performance may be expected when using the number of features forming a local maximum. As a result of the RFECV analysis, when four features were used, the local maximum was formed (FIG. 4A and 4B). The selected four features are hsa_miR_125b_5p, hsa_miR_141_5p, hsa_miR_17_3p, and hsa_miR_30c_5p. A subsequent machine learning-based study was conducted using the four-miRNA combination with high feature importance, and a study including PCA3_4 and TMPRSS2:ERG Fusion_R3, which are two additional features that had been already widely studied, as well as the four selected miRNAs, was also conducted. The PCA3_4 and TMPRSS2:ERG also exhibited high feature importance. By the feature selection process, the four-miRNA combination (the combination of four features) and a combination formed by adding one lncRNA and one mRNA of a fusion gene to the four miRNAs (combination of six features) were selected for machine learning (ML). In addition, the four-miRNA combination selected as above and combinations made by adding one lncRNA and one mRNA of a fusion gene thereto were used for the conventional statistical analysis method. This method is merely an example of the method of selecting a biomarker, and thus the present invention is not limited thereto.

**Machine learning based on single biomarker**

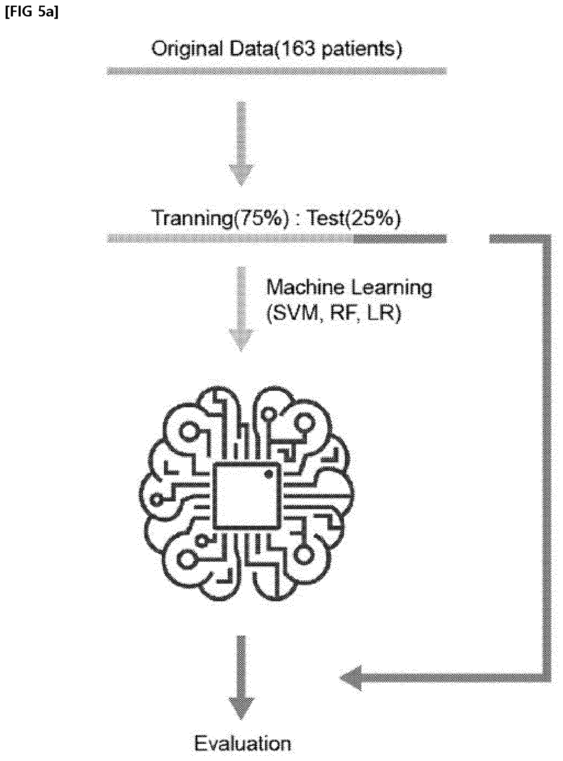

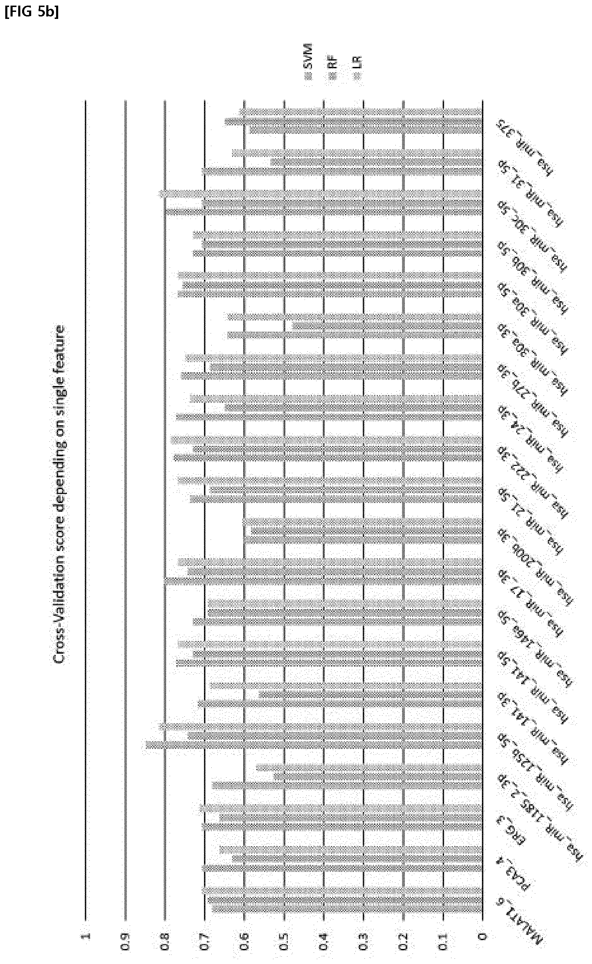

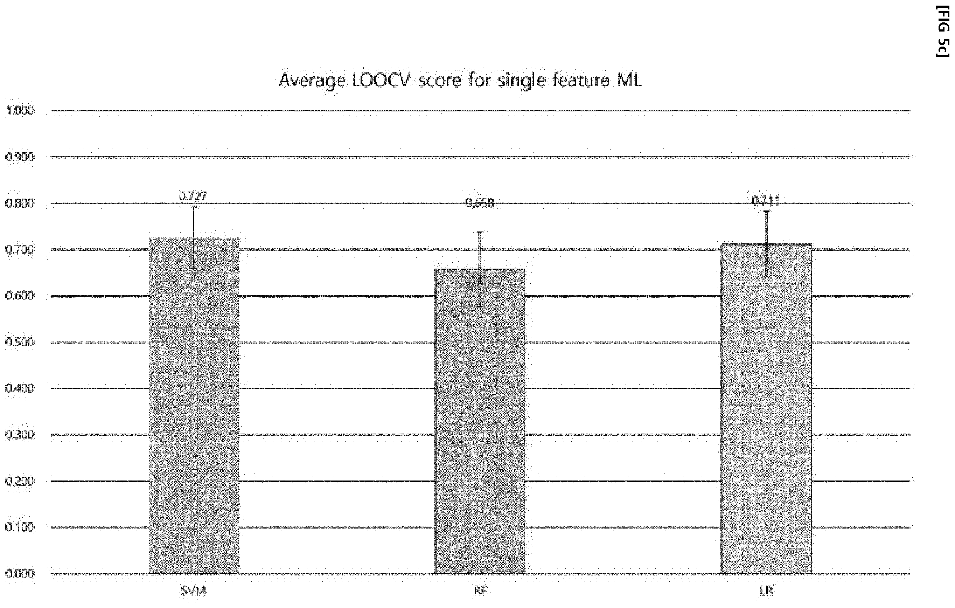

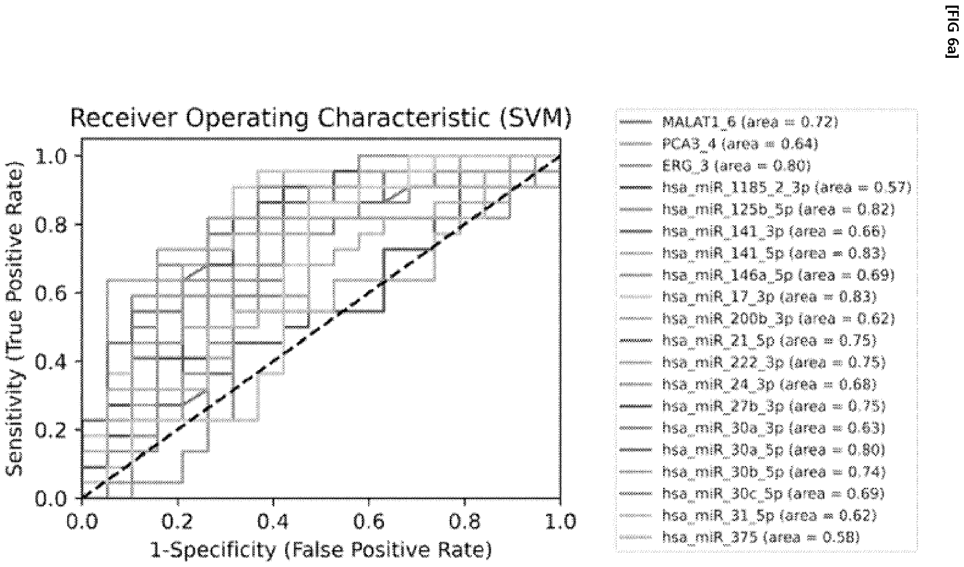

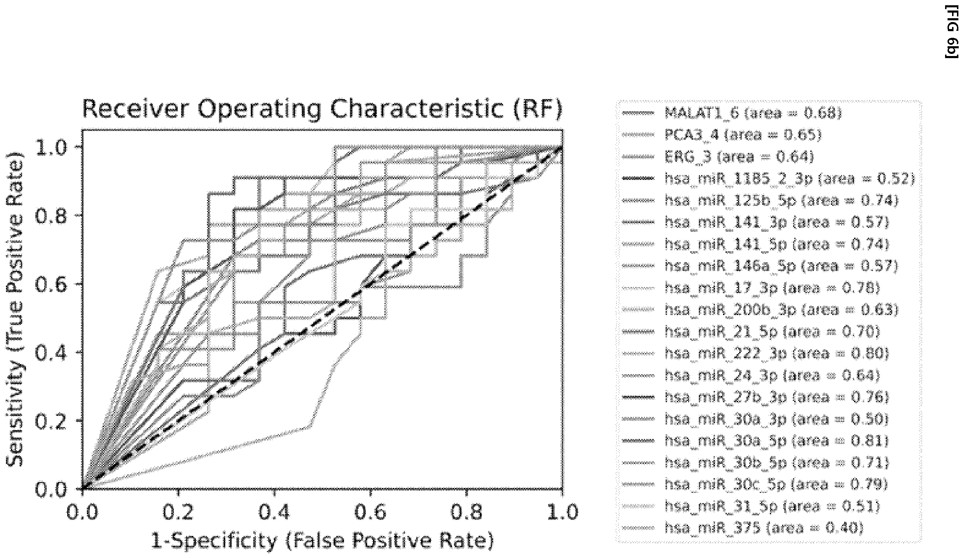

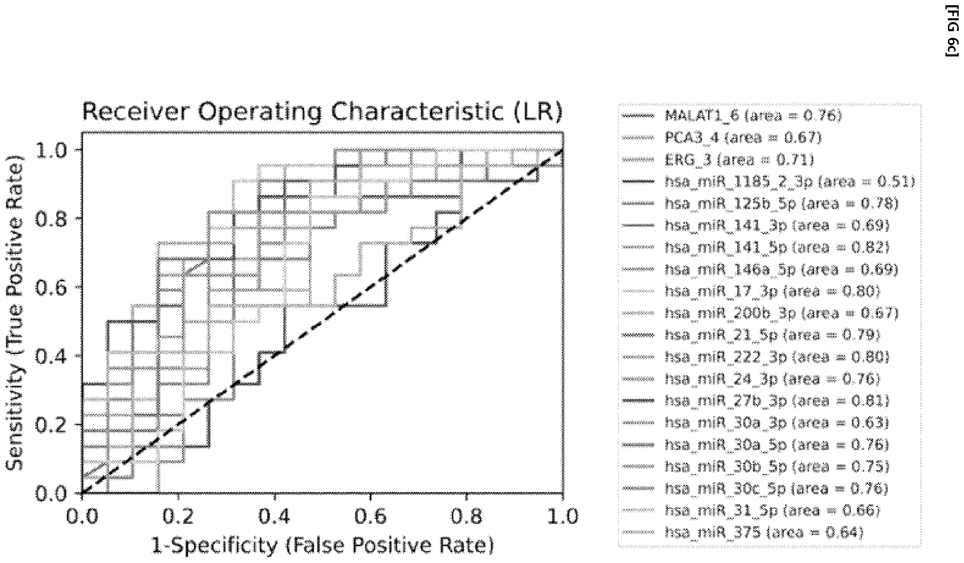

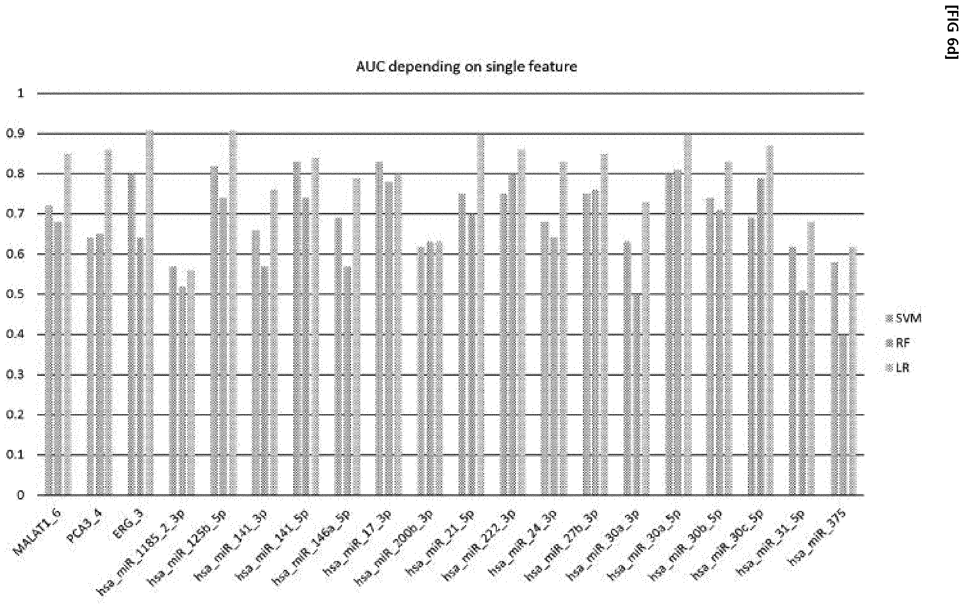

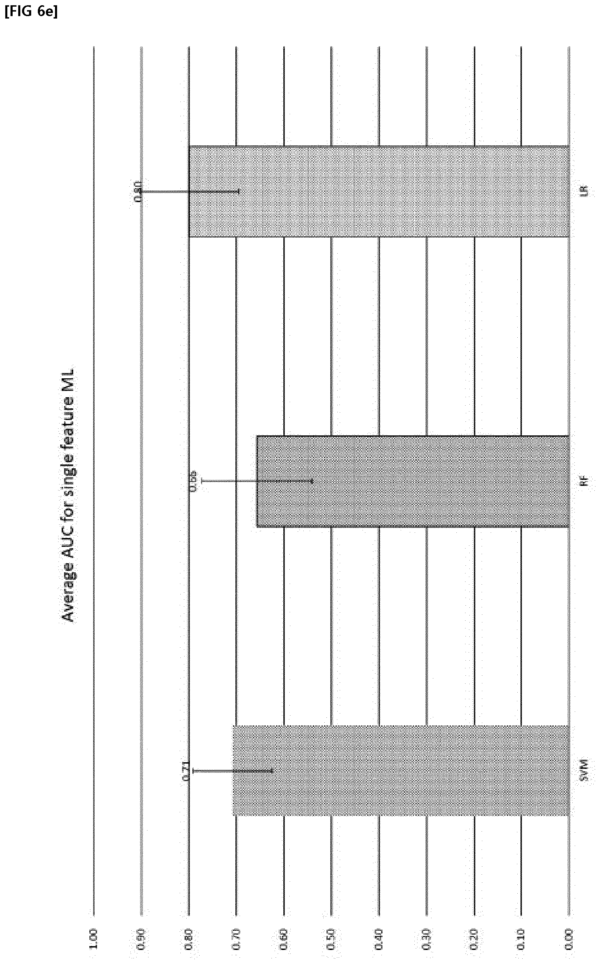

To control multi-biomarker analysis, a single biomarker-based diagnostic classifier was developed using the ML algorithm. First, the min-max scaled-data was randomly divided into a training set (75%) and a data set (25%). After the training-test set division, a support vector machine (SVM), a random forest (RF), and a logistic regression (LR) algorithm were trained using the training set (FIG. 2). Due to the numerical imbalance between PCa and BPH, class weight compensation was performed in all three algorithms. After the training set learning, an ROC curve, and AUC and LOOCV scores for all biomarkers and ML algorithms were generated (FIGS. 5B and 5C, and FIGS. 6A to 6E)

Depending on the biomarker, each machine learning algorithm showed significant differences in AUC and LOOCV scores. In the order of SVM, RF, and LR, the average AUC values were 0.71±0.08 (standard deviation), 0.66±0.12, and 0.80±0.10, and the average LOOCV values were 0.727±0.066, 0.658±0.080, and 0.711±0.071. According to these results, SVM and LR are more suitable than RF for single biomarker-based ML classification. However, the overall trend of the AUC and LOOCV scores seems to be greatly influenced by the biomarkers, rather than the ML algorithms. Since the above result is based on a single biomarker, when the same analysis was applied to multiple biomarkers, a better result is expected due to a synergy of the combination of biomarkers.

**Machine learning based on multiple biomarkers**

A multi-biomarker-based PCa/BPH classifier was created with the goal of improving performance and robustness. Data preprocessing and machine learning algorithm learning were performed in the same manner as those for the single biomarker-based classifier, and to achieve the previously proposed goal, parameter optimization was performed through the GridSearchCV algorithm for each biomarker combination and algorithm.

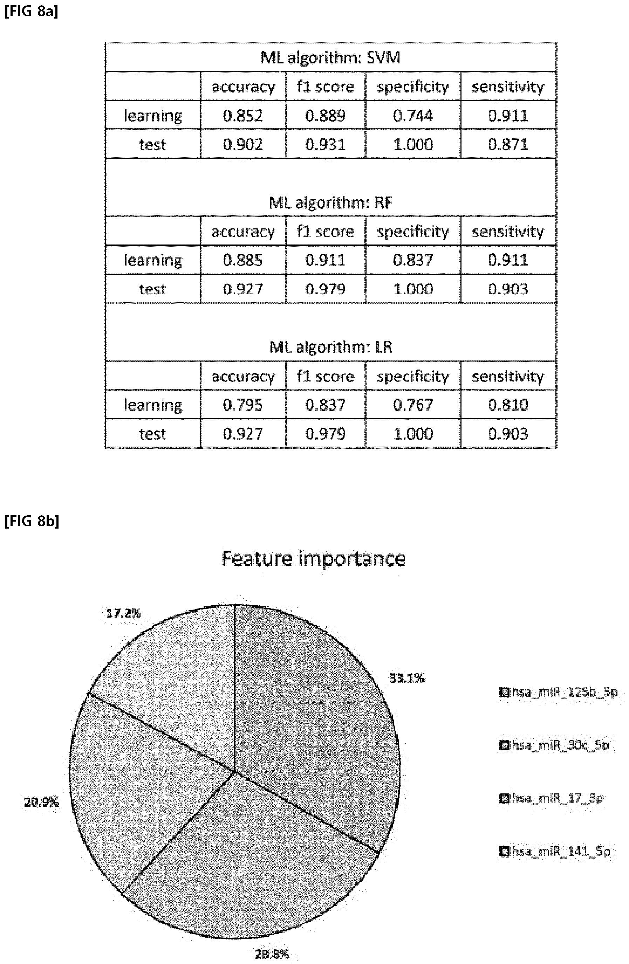

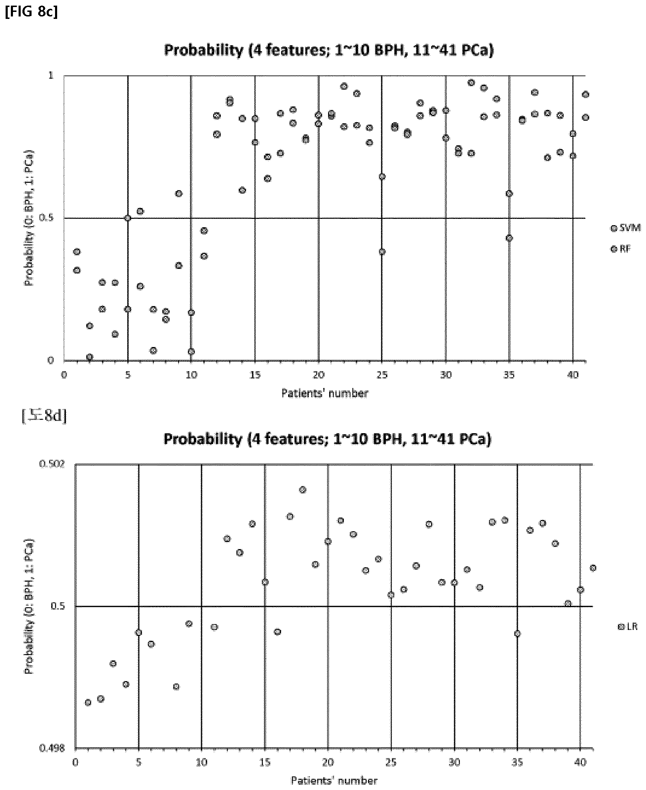

As a result of machine learning, for the combination of four miRNAs, in the order of SVM, RF, and LR, the AUC values were 0.96, 0.99, and 0.96 (FIGS. 7C to 7E), and the LOOCV values were 0.847, 0.865, and 0.834 (FIG. 7A). In terms of feature importance, hsa_miR_125b_5p was 33.1%, hsa_miR_30c_5p was 28.8%, hsa_miR_17_3p was 20.9%, and hsa_miR_141_5p was 17.2% (FIG. 8B). In the analysis targeting a patient group with PSA levels of 3 to 10, in the order of SVM, RF, and LR, it was confirmed that the AUC levels were 0.94, 0.99, and 0.91, and the LOOCV values were 0.885, 0.896, and 0.854. Leave-one-out cross-validation (LOOCV) is a method of verifying a total of n models through modeling using n-1 samples as a training set, excluding only one sample from the total of n samples, and according to this, randomness depending on a sample may be removed, thereby increasing the accuracy of prediction.

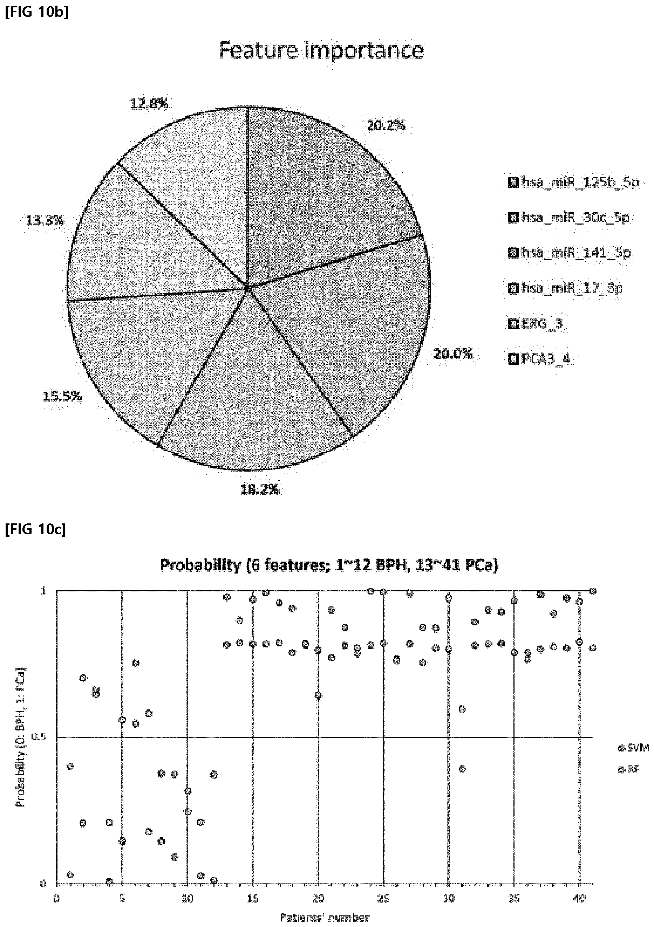

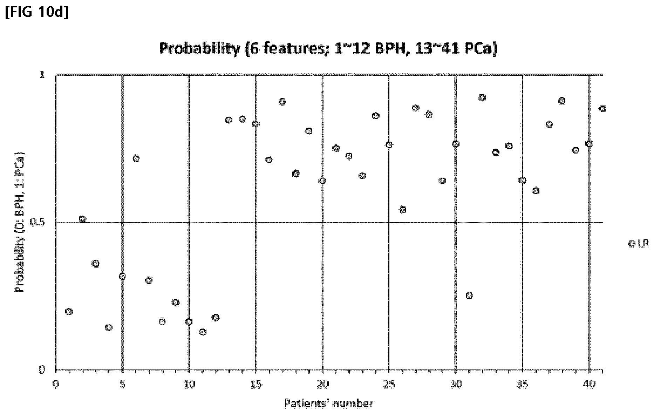

In the case of the combination of six biomarkers, in the order of SVM, RF, and LR, the AUC values were 0.96, 0.99, and 0.96 (FIGS. 9C to 9E), and the LOOCV values were 0.865, 0.871, and 0.834 (FIG. 9A). In terms of feature importance, hsa_miR_125b_5p was 20.2%, hsa_miR_30c_5p was 20.0%, hsa_miR_141_5p was 18.2%, hsa_miR_17_3p was 15.5%, TMPRSS2:ERG Fusion_R3 was 13.3%, and PCA3_4 was 12.8% (FIG. 10B). In the case of feature importance, as predicted by RFECV, miRNAs were found to account for more than half (73.9%). In the analysis targeting the patient group with PSA levels of 3 to 10, in the order of SVM, RF, and LR, it was confirmed that the AUC values were 0.94, 1.00, and 0.93, and the LOOCV values were 0.865, 0.896, and 0.844.

Considering the above results, it is expected that the multi-biomarker-based machine learning classifier using the miRNA combination or IncRNA, fusion gene mRNA, and miRNA, proposed in this study, has higher diagnosis performance than that of the conventional test using conventional miRNA, IncRNA, fusion gene mRNA, and PSA. Particularly, considering that the importance of each miRNA was relatively equal as a result of random forest learning, it seems that the significant performance improvement observed in the multi-biomarker classifier is caused by the synergistic effect between features. In addition, since the multi-biomarker-based machine learning classifier proposed in this study effectively classifies the patient group corresponding to the gray zone (PSA level of 3 to 10) in the PSA test, it may be widely used as a complement to the preexisting diagnosis method. Moreover, it may contribute to patient-customized treatment in diagnosis and treatment processes by providing probability beyond single binary classification. As the multi-biomarker classifier uses modern machine learning techniques, it is expected to be expanded in many ways by combining with other assays and biomarkers in the future.

**Conventional statistical analysis method based on multiple biomarkers**

Separate from the multi-biomarker-based machine learning method, a multi-biomarker-based diagnostic classifier was developed using a conventional analysis method. In the multi-biomarker-based statistical analysis method, data preprocessing, statistical analysis algorithm, parameters, and evaluation methods are the same as those in the single biomarker-based machine learning. When logistic regression, which is one of the conventional statistical analysis methods, is used, values vary depending on the combination of multiple biomarkers, but the maximum AUC value is 0.90, and the maximum LOOCV value is 0.88. In comparison with the single biomarker classifier by machine learning, significant improvements in AUC and LOOCV values were observed. Like the multi-biomarker-based machine learning, when using the conventional multi-biomarker-based statistical analysis method, performance and robustness may be improved, compared with the single biomarker-based machine learning method. While the method of selecting a biomarker is the same as described above, in the conventional statistical analysis method, such as logistic regression, not only the combination of the selected 4 miRNAs, the four miRNAs, one lncRNA, one mRNA of a fusion gene, but also all combinations consisting of this group may be used. The AUC and LOOCV values were compared using the all combinations of multiple biomarkers that could be grouped, and by determining the ranking according to the AUC and LOOCV values and adding the AUC and LOOCV ranks, the next highest combination may be derived and used starting from the combination with the lowest sum, that is, the highest ranking of the sum. The method of forming a combination or the number of combinations may vary depending on the method of reflecting the importance of performance and robustness of the classifier, and the finally applied biomarker combination may be selected from the next highest rank combination, but the present invention is not limited thereto.

Scores for distinguishing a PCa patient from a BPH patient through logistic regression may be calculated, and a method of calculating a score is as follows.

1) For an individual biomarker, the maximum expression level of an experimental group is normalized to 1, and the minimum expression level is normalized to 0.

2) The decision function (D(x)) thereof is calculated as follows.

$$D(x) = bias + sum(coefficient * normalized\ value)$$

3) A sigmoid function (F(x)) is calculated based on a decision function value, thereby obtaining the final score.

$$F(x) = 1/(1+exp(-D(x)))$$

4) The final score is evaluated based on a threshold of 0.5, and a score of 0.5 or higher is judged to be prostate cancer, and a score of less than 0.5 is judged to be benign prostate hyperplasia.

AUC and LOOCV values according to the bias and coefficient, which were used for each biomarker, and their combination may be obtained and then compared with the combinations of biomarkers showing high performance through the logistic regression technique.

**Tools for statistical analysis and machine learning**

All statistical analyses were performed by R (ver. 4.0.2) and Python (ver. 3.9).

(1) Data preprocessing (Data blank and normalization)

The preprocessing of raw data was performed using dplyr, reshape2 and tidyr package of R (ver. 4.0.2). The No Ct value of the raw data was treated as a blank, and the expression levels of lncRNA, miRNA, and mRNA of a fusion gene were normalized with the average of $\beta$-actin and 18s-rRNA. The normalization was performed as follows:

$$Normalized\ expression\ (-\Delta Ct) = (Ct_{\beta\text{-actin}} + Ct_{18s\text{-rRNA}})/2 - Ct_{target\ gene}$$

After normalization, blank normalized expression was treated as the minimum normalized expression in the same biomarker.

(2) Single marker analysis

Single marker analysis was performed using the ggplot2 and plotROC package of R (ver. 4.0.2). For single marker analysis, the previously processed normalized expression (-$\Delta$Ct) of biomarkers for lncRNA, miRNA, and mRNA of a fusion gene was used. To confirm the gene expression trends between two groups ((BPH) and PCa)), the normalized expression (-$\Delta$Ct) of each biomarker was visualized as a box plot. In addition, ROC curve analysis was performed to evaluate the differential ability of each biomarker.

**Construction of PCa/BPH classifier through machine learning algorithm**

To produce a PCa/BPH classifier based on a single biomarker or multiple biomarkers, customized machine learning software using Python 3.8.5 was run on Linux. The software includes open libraries such as Scikit Learn, Scikit Plot, and Panda. For a support vector machine, C-Support Vector Classification (Scikit Learn) was used. A radial basis function was used as a kernel, and a balanced class weight was used considering the numerical difference between classes. For RF, a random forest classifier (Scikit Learn) was used, and the model consists of 100 decision trees with balanced class weights. Finally, logistic regression was also imported from Scikit Learn, and all parameters except balanced class weights followed the default settings.

Before introduction of qPCR data into the machine learning algorithm, data preprocessing was performed. First, under the assumption that the amount of lncRNA, miRNA, or mRNA of a fusion gene is very small, as described above, non-detected CT values were filled with the minimum value of the biomarker. A min-max scaler was applied to satisfied qPCR data to normalize the CT value between 0 and 1. The normalized data was randomly divided into a training set (75%) and a test set (25%). Each machine learning algorithm was trained with the training set, and model accuracy, an f1 score, a receiver operating characteristic (ROC) curve, AUC, and probability were evaluated using the test set. To check for overfitting problems, cross-validation was also provided. Particularly, to avoid potential group bias, the leave one out algorithm was applied.

## 2. Results

To date, much research has been conducted on prostate cancer-specific biomarkers, but in the case of localized prostate cancer, it was reported that the sensitivity and specificity in the detection of a biomarker in body fluids were very low, and a prostate-specific antigen (PSA) is widely used to detect prostate cancer, but since it is not a cancer-specific marker, it is often misdiagnosed. Since the prostate tissue biopsy performed for an accurate examination causes severe pain to a patient and high costs, a non-invasive biomarker for early discovery of prostate cancer was needed.

Therefore, to verify the usefulness of the prostate cancer lncRNA, miRNA, and mRNA of a fusion gene markers of the present invention, as a result of comparing the expression levels of lncRNA, miRNA, and mRNA of a fusion gene in the BPH control and PCa, as shown in FIG. 2, box plots showed that both the lncRNA and mRNA markers were up-expressed and all of the miRNA markers were down-expressed in the total prostate cancer urine samples.

In addition, it was proved that the combination of four miRNAs (hsa_miR_125b_5p, hsa_miR_141_5p, hsa_miR_17_3p, and hsa_miR_30c_5p) deduced from random forest analysis using the RFECV algorithm was useful particularly as a detection marker, and the combination in which one lncRNA (PCA3) and one mRNA of a fusion gene (TMPRSS2-ERG) were added to the four-miRNA combination was also useful as a detection marker.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as being illustrative and not restrictive in any aspect.

[Explanation of abbreviations]

miRNA: microRNA
PCa: prostate cancer
BPH: benign prostatic hyperplasia
PSA: prostate-specific antigen

## Claims

1. A composition for diagnosing prostate cancer, comprising:
   an agent that can measure an expression level of miRNA, which includes hsa_miR_125b_5p, hsa_miR_17_3p, hsa_miR_141_5p, and hsa_miR_30c_5p.

2. The composition of claim 1, wherein the miRNA further includes at least one selected from the group consisting of hsa_miR_21_5p, hsa_miR_24_3p, hsa_miR_30a_5p, hsa_miR_222_3p, hsa_miR_375, hsa_miR_27b_3p, hsa_miR_31_5p, hsa_miR_146a_3p, hsa_miR_146a_5p, hsa_miR_200b_3p, hsa_miR_1185_2_3p, hsa_miR_30a_3p, and hsa_miR_30b_5p.

3. The composition of claim 1, further comprising at least one selected from the group consisting of lncRNA and mRNA of a fusion gene, wherein the lncRNA is at least one selected from PCA3 and MALAT1, and the mRNA of a fusion gene is TMPRSS2:ERG.

4. The composition of claim 3, wherein the lncRNA is PCA3.

5. The composition of claim 1, wherein the miRNA further includes at least one selected from the group consisting of hsa_miR_21_5p, hsa_miR_24_3p, hsa_miR_30a_5p, and hsa_miR_222_3p.

6. The composition of any one of claims 1, 2, and 5, wherein the agent includes a primer or probe that specifically binds to each miRNA.

7. The composition of claim 6, wherein the miRNA consists of a base sequence listed in the table below:

| miRNA | Sequence(5' → 3') |
|---|---|
| hsa-miR-1185-2-3p | AUAUACAGGGGGAGACUCUCAU (SEQ ID NO: 19) |
| hsa-miR-125b-5p | UCCCUGAGACCCUAACUUGUGA (SEQ ID NO: 20) |
| hsa-miR-141-3p | UAACACUGUCUGGUAAAGAUGG (SEQ ID NO: 21) |
| hsa-miR-141-5p | CAUCUUCCAGUACAGUGUUGGA (SEQ ID NO: 22) |
| hsa-miR-146a-5p | UGAGAACUGAAUUCCAUGGGUU (SEQ ID NO: 23) |
| hsa-miR-17-3p | ACUGCAGUGAAGGCACUUGUAG (SEQ ID NO: 24) |
| hsa-miR-200b-3p | UAAUACUGCCUGGUAAUGAUGA (SEQ ID NO: 25) |
| hsa-miR-21-5p | UAGCUUAUCAGACUGAUGUUGA (SEQ ID NO: 26) |
| hsa-miR-222-3p | AGCUACAUCUGGCUACUGGGU (SEQ ID NO: 27) |
| hsa-miR-24-3p | UGGCUCAGUUCAGCAGGAACAG (SEQ ID NO: 28) |
| hsa-miR-27b-3p | UUCACAGUGGCUAAGUUCUGC (SEQ ID NO: 29) |
| hsa-miR-30a-3p | CUUUCAGUCGGAUGUUUGCAGC (SEQ ID NO: 30) |
| hsa-miR-30a-5p | UGUAAACAUCCUCGACUGGAAG (SEQ ID NO: 31) |
| hsa-miR-30b-5p | UGUAAACAUCCUACACUCAGCU (SEQ ID NO: 32) |
| hsa-miR-30c-5p | UGUAAACAUCCUACACUCUCAGC (SEQ ID NO: 33) |
| hsa-miR-31-5p | AGGCAAGAUGCUGGCAUAGCU (SEQ ID NO: 34) |
| hsa-mir-375 | CCCCGCGACGAGCCCCUCGCACAAACCGGACCUGAGCGUUUUGUUCGUUCGGCUC GCGUGAGGC (SEQ ID NO: 35) |

8. The composition of claim 3 or 4, wherein the agent includes a primer or probe that specifically binds to miRNA, lncRNA, or mRNA of a fusion gene.

9. The composition of claim 8, wherein the primer that specifically binds to miRNA, lncRNA, or mRNA of a fusion gene consists of a base sequence listed in the tables below:

| Primers of lncRNA or mRNA of fusion gene | Forward(5' → 3') | Reverse(5' → 3') |
|---|---|---|
| PCA3_4 | GAGAACAGGGGAGGGAGAG (SEQ ID NO: 1) | ACGTTCTGGGATACATGTGC (SEQ ID NO: 2) |

(continued)

| Primers of lncRNA or mRNA of fusion gene | Forward(5' → 3') | Reverse(5' → 3') |
|---|---|---|
| PCA3_C2 | TGATAGGTGCAGCAAACCAC (SEQ ID NO: 3) | ACGTTCTGGGATACATGTGCAG (SEQ ID NO: 4) |
| TMPRSS2:ERG_R3 | CCTGGAGCGCGGCAGGAAGCCTTAT CAGTTG (SEQ ID NO: 5) | TCCTGCTGAGGGACGCGTGGGCTC ATCTTG (SEQ ID NO: 6) |
| TMPRSS2:ERG_R6 | GGCAGGAACTCTCCTGAT (SEQ ID NO: 7) | CGTGGCACGATAACTCTG (SEQ ID NO: 8) |

| miRNA | Sequence(5' → 3') |
|---|---|
| hsa-miR-1185-2-3p | AUAUACAGGGGGAGACUCUCAU (SEQ ID NO: 19) |
| hsa-miR-125b-5p | UCCCUGAGACCCUAACUUGUGA (SEQ ID NO: 20) |
| hsa-miR-141-3p | UAACACUGUCUGGUAAAGAUGG (SEQ ID NO: 21) |
| hsa-miR-141-5p | CAUCUUCCAGUACAGUGUUGGA (SEQ ID NO: 22) |
| hsa-miR-146a-5p | UGAGAACUGAAUUCCRLIGGGUU (SEQ ID NO: 23) |
| hsa-miR-17-3p | ACUGCAGUGAAGGCACUUGUAG (SEQ ID NO: 24) |
| hsa-miR-200b-3p | UAAUACUGCCUGGUAAUGAUGA (SEQ ID NO: 25) |
| hsa-miR-21-5p | UAGCUUAUCAGACUGAUGUUGA (SEQ ID NO: 26) |
| hsa-miR-222-3p | AGCUACAUCUGGCUACUGGGU (SEQ ID NO: 27) |
| hsa-miR-24-3p | UGGCUCAGUUCAGCAGGAACAG (SEQ ID NO: 28) |
| hsa-miR-27b-3p | UUCACAGUGGCUAAGUUCUGC (SEQ ID NO: 29) |
| hsa-miR-30a-3p | CUUUCAGUCGGAUGUUUGCAGC (SEQ ID NO:30) |
| hsa-miR-30a-5p | UGUAAACAUCCUCGACUGGAAG (SEQ ID NO: 31) |
| hsa-miR-30b-5p | UGUAAACAUCCUACACUCAGCU (SEQ ID NO: 32) |
| hsa-miR-30c-5p | UGUAAACAUCCUACACUCUCAGC (SEQ ID NO: 33) |
| hsa-miR-31-5p | AGGCAAGAUGCUGGCAUAGCU (SEQ ID NO: 34) |
| hsa-mir-375 | CCCCGCGACGAGCCCCUCGCACAAACCGGACCUGAGCGUUUUGUUCGUUCGGCUC GCGUGAGGC (SEQ ID NO: 35) |

10. A method of providing information for diagnosing prostate cancer, comprising:

measuring an expression level of miRNA of any one of claims 1, 2, and 5 in a biological sample isolated from a subject; and
comparing the expression level of the miRNA with an expression level of miRNA of a normal control sample.

11. The method of claim 10, wherein the expression level of the miRNA is measured by reverse transcriptase polymerase chain reaction (RT-PCR), competitive RT-PCR, quantitative RT-PCR (qPCR), droplet digital PCR (ddPCR), sequencing, an RNase protection method, Northern blotting, and/or gene chip analysis.

12. The method of claim 10, wherein the measuring of an expression level comprises:

extracting the miRNA from a biological sample isolated from a subject; synthesizing cDNA from the extracted miRNA; amplifying the cDNA using a primer pair specific for the synthesized cDNA; and detecting the amplified cDNA.

13. The method of claim 10, wherein the biological sample is tissue, cells, urine, saliva, semen, whole blood, plasma, or serum.

14. The method of claim 13, wherein the biological sample is urine.

15. The method of claim 10, wherein the comparing of the expression level of the miRNA is performed using a machine learning algorithm selected from the group consisting of logistic regression (LR), a support vector machine (SVM), a random forest, and a multi-layer perceptron.

16. The method of claim 10, wherein the comparing of the expression level of the miRNA is performed using logistic regression (LR), a leave-one-out cross-validation (LOOCV) value is 0.65 or more, and an area under the curve (AUC) value is 0.70 or more.

17. The method of claim 10, wherein the measuring of an expression level further comprises measuring the expression of lncRNA, mRNA of a fusion gene, or a combination thereof, in which the lncRNA is at least one selected from PCA3 and MALAT1, and the mRNA of a fusion gene is TMPRSS2:ERG, and the comparing of the expression level further comprises comparing the expression level of the lncRNA, mRNA of a fusion gene, or a combination thereof with that of lncRNA, mRNA of a fusion gene, or a combination thereof in a normal control sample.

18. The method of claim 17, wherein the expression level of the miRNA, lncRNA, mRNA of a fusion gene, or a combination thereof is measured by reverse transcriptase polymerase chain reaction (RT-PCR), competitive RT-PCR, quantitative RT-PCR (qPCR), droplet digital PCR (ddPCR), sequencing, an RNase protection method, Northern blotting, or gene chip analysis.

19. The method of claim 17, wherein the measuring of an expression level comprises extracting the miRNA, lncRNAs including PCA3 and MALAT1, mRNA of a fusion gene including TMPRSS2:ERG, or a combination thereof from a biological sample isolated from a subject; synthesizing cDNA from the extracted miRNA, lncRNAs and mRNA of a fusion gene, or combination thereof; amplifying the cDNA using a primer set specific for the synthesized cDNA; and detecting the amplified cDNA.

20. The method of claim 17, wherein the biological sample is tissue, cells, urine, saliva, semen, whole blood, plasma, or serum.

21. The method of claim 20, wherein the biological sample is urine.

22. The method of claim 17, wherein the measuring of the expression of miRNA with that of lncRNA, mRNA of a fusion gene, or a combination thereof is performed using a machine learning algorithm selected from the group consisting of logistic regression (LR), a support vector machine (SVM), a random forest, and a multi-layer perceptron.

23. The method of claim 17, wherein the comparing of the expression level of the miRNA with that of lncRNA, mRNA of a fusion gene, or a combination thereof is performed using logistic regression (LR), a leave-one-out cross-validation (LOOCV) value is 0.65 or more, and an area under the curve (AUC) value is 0.70 or more.

24. A kit for diagnosing prostate cancer, comprising the composition of any one of claims 1 to 5.

[FIG 1]

[FIG 2a]

[FIG 2b]

**[FIG 2c]**

[FIG 2d]

[FIG 2e]

[FIG 2f]

[FIG 2g]

[FIG 2h]

[FIG 3a]

[FIG 3b]

[FIG 3c]

[FIG 3d]

[FIG 3e]

[FIG 3f]

[FIG 3g]

[FIG 3h]

[FIG 4a]

RFECV, RF(150), LOO accuracy, all data

[FIG 4b]

| Feature | Rank |
|---|---|
| hsa_miR_125b_5p | 1 |
| hsa_miR_141_5p | 1 |
| hsa_miR_17_3p | 1 |
| hsa_miR_30c_5p | 1 |
| ERG_3 | 2 |
| hsa_miR_222_3p | 3 |
| hsa_miR_30a_5p | 4 |
| hsa_miR_24_3p | 5 |
| PCA3_4 | 6 |
| hsa_miR_21_5p | 7 |
| hsa_miR_27b_3p | 8 |
| hsa_miR_30a_3p | 9 |
| MALAT1_6 | 10 |
| hsa_miR_30b_5p | 11 |
| hsa_miR_31_5p | 12 |
| hsa_miR_1185_2_3p | 13 |
| hsa_miR_200b_3p | 14 |
| hsa_miR_141_3p | 15 |
| hsa_miR_146a_5p | 16 |
| hsa_miR_375 | 17 |

[FIG 5a]

Original Data(163 patients)

Tranning(75%) : Test(25%)

Machine Learning
(SVM, RF, LR)

Evaluation

[FIG 5b]

Cross-Validation score depending on single feature

[FIG 5c]

Average LOOCV score for single feature ML

EP 4 365 310 A1

Receiver Operating Characteristic (SVM)

MALAT1_6 (area = 0.72)
PCA3_4 (area = 0.64)
ERG_3 (area = 0.80)
hsa_miR_1185_2_3p (area = 0.57)
hsa_miR_125b_5p (area = 0.82)
hsa_miR_141_3p (area = 0.66)
hsa_miR_141_5p (area = 0.83)
hsa_miR_146a_5p (area = 0.69)
hsa_miR_17_3p (area = 0.83)
hsa_miR_200b_3p (area = 0.62)
hsa_miR_21_5p (area = 0.75)
hsa_miR_222_3p (area = 0.75)
hsa_miR_24_3p (area = 0.68)
hsa_miR_27b_3p (area = 0.75)
hsa_miR_30a_3p (area = 0.63)
hsa_miR_30a_5p (area = 0.80)
hsa_miR_30b_5p (area = 0.74)
hsa_miR_30c_5p (area = 0.69)
hsa_miR_31_5p (area = 0.62)
hsa_miR_375 (area = 0.58)

[FIG 6b]

[FIG 6c]

[FIG 6d]

AUC depending on single feature

[FIG 6e]

Average AUC for single feature ML

[FIG 7a]

LOOCV score comparison (4 features)

[FIG 7b]

- **Prediction**

Real:               [1 1 0 0 1 1 1 1 1 1 0 1 1 0 1 1 0 1 1 0 1 1 1 1 1 1 1 1 0 0 0 1 1 1 0 1]

SVM prediction:  [**0** 1 0 0 1 1 1 **0** 1 1 1 0 1 1 1 0 1 1 0 **0** 1 1 0 1 1 1 1 1 1 0 1 1 0 0 0 1 1 1 0 1]

RF prediction:    [**0** 1 0 0 1 1 1 1 1 1 0 1 1 1 0 1 1 0 **0** 1 1 0 1 1 1 1 1 1 0 1 1 0 0 0 1 1 1 0 1]

LR prediction:    [**0** 1 0 0 1 1 1 **0** 1 1 1 0 1 1 1 0 1 1 0 1 1 1 0 1 1 1 1 1 0 1 1 0 0 0 1 1 1 0 1]

[FIG 7c]

**[FIG 7d]**

RF ROC

**[FIG 7e]**

LR ROC

[FIG 8a]

| ML algorithm: SVM | | | | |
|---|---|---|---|---|
| | accuracy | f1 score | specificity | sensitivity |
| learning | 0.852 | 0.889 | 0.744 | 0.911 |
| test | 0.902 | 0.931 | 1.000 | 0.871 |

| ML algorithm: RF | | | | |
|---|---|---|---|---|
| | accuracy | f1 score | specificity | sensitivity |
| learning | 0.885 | 0.911 | 0.837 | 0.911 |
| test | 0.927 | 0.979 | 1.000 | 0.903 |

| ML algorithm: LR | | | | |
|---|---|---|---|---|
| | accuracy | f1 score | specificity | sensitivity |
| learning | 0.795 | 0.837 | 0.767 | 0.810 |
| test | 0.927 | 0.979 | 1.000 | 0.903 |

[FIG 8b]

Feature importance

17.2%  33.1%  20.9%  28.8%

hsa_miR_125b_5p

hsa_miR_30c_5p

hsa_miR_17_3p

hsa_miR_141_5p

[FIG 8c]

Probability (4 features; 1~10 BPH, 11~41 PCa)

[도 8d]

Probability (4 features; 1~10 BPH, 11~41 PCa)

[FIG 9a]

LOOCV score comparison (6 features)

[FIG 9b]

- **Prediction**

Real:            [1 1 0 1 1 1 1 0 1 1 1 0 1 1 1 0 1 0 1 1 0 1 0 1 0 1 0 1 1 1 1 0 1 1 1 1 0 1]
SVM prediction:  [1 1 0 1 1 1 1 1 1 1 1 1 1 1 1 0 1 0 1 1 0 1 0 **0** 0 1 1 1 1 1 1 0 1 1 1 0 1]
RF prediction:   [1 1 0 1 1 1 1 0 1 1 1 **1** 1 1 1 0 1 0 1 1 0 1 0 **0** 0 1 1 1 0 1 1 0 1 1 1 0 1]
LR prediction:   [1 1 0 1 1 1 1 1 1 1 1 1 1 1 1 0 1 0 1 1 0 1 0 **0** 0 1 1 1 1 1 1 0 1 1 1 0 1]

[FIG 9c]

SVM ROC for PSA 3~10

[FIG 9d]

RF ROC for PSA 3~10

[FIG 9e]

LR ROC for PSA 3~10

[FIG 10a]

| ML algorithm: SVM | | | |
|---|---|---|---|
| | accuracy | f1 score | specificity | sensitivity |
| learning | 0.836 | 0.878 | 0.732 | 0.889 |
| test | 0.927 | 0.949 | 0.833 | 0.966 |

| ML algorithm: RF | | | |
|---|---|---|---|
| | accuracy | f1 score | specificity | sensitivity |
| learning | 1.000 | 1.000 | 1.000 | 1.000 |
| test | 0.927 | 0.949 | 0.833 | 0.966 |

| ML algorithm: LR | | | |
|---|---|---|---|
| | accuracy | f1 score | specificity | sensitivity |
| learning | 0.787 | 0.831 | 0.780 | 0.790 |
| test | 0.927 | 0.979 | 0.833 | 0.966 |

[FIG 10b]

## Feature importance

12.8%

20.2%

13.3%

☒ hsa_miR_125b_5p

☒ hsa_miR_30c_5p

☒ hsa_miR_141_5p

☒ hsa_miR_17_3p

☒ ERG_3

☐ PCA3_4

20.0%

15.5%

18.2%

[FIG 10c]

## Probability (6 features; 1~12 BPH, 13~41 PCa)

Probability (0: BPH, 1: PCa)

⊙ SVM
⊙ RF

Patients' number

[FIG 10d]

Probability (6 features; 1~12 BPH, 13~41 PCa)

### INTERNATIONAL SEARCH REPORT

| International application No. |
| --- |
| **PCT/KR2022/007695** |

**A. CLASSIFICATION OF SUBJECT MATTER**

**C12Q 1/6886**(2018.01)i

According to International Patent Classification (IPC) or to both national classification and IPC

**B. FIELDS SEARCHED**

Minimum documentation searched (classification system followed by classification symbols)

C12Q 1/6886(2018.01); C12N 15/11(2006.01); C12P 19/34(2006.01); C12Q 1/68(2006.01); G01N 33/574(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 전립선암(prostate cancer), 진단(diagnosis), has_miR_125b_5p, has-miR_17_3p, has_miR-141-5p, has_miR_30c_5p

**C. DOCUMENTS CONSIDERED TO BE RELEVANT**

| Category* | Citation of document, with indication, where appropriate, of the relevant passages | Relevant to claim No. |
| --- | --- | --- |
| Y | MARTIGNANO, F. et al. Urinary RNA-based biomarkers for prostate cancer detection. Clinica Chimica Acta. 2017, vol. 473, pp. 96-105. <br> See abstract; table 1; and pages 97-98. | 1-24 |
| Y | FREDSØE, J. et al. Diagnostic and prognostic microRNA biomarkers for prostate cancer in cell-free urine. European Urology Focus. 2018, vol. 4, pp. 825-833. <br> See abstract; table 2; and pages 827 and 829-831. | 1-24 |
| Y | URABE, F. et al. Large-scale Circulating microRNA Profiling for the Liquid Biopsy of Prostate CancerSerum miRNA Biomarker for Prostate Cancer. Clinical Cancer Research. 2019, vol. 25, no. 10, pp. 3016-3025. <br> See abstract; and pages 3019 and 3022-3024. | 1-24 |
| A | US 10822661 B2 (MIODX) 03 November 2020 (2020-11-03) <br> See abstract; and claims 1-20. | 1-24 |
| A | KR 10-2013-0043104 A (CARIS LIFE SCIENCES LUXEMBOURG HOLDINGS) 29 April 2013 (2013-04-29) <br> See abstract; and claims 1, 3 and 71. | 1-24 |

☐ Further documents are listed in the continuation of Box C.   ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **30 August 2022** | **31 August 2022** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/007695** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.   ☑   forming part of the international application as filed:

          ☑   in the form of an Annex C/ST.25 text file.

          ☐   on paper or in the form of an image file.

    b.   ☐   furnished together with the international application under PCT Rule 13*ter*.1(a) for the purposes of international search only in the form of an Annex C/ST.25 text file.

    c.   ☐   furnished subsequent to the international filing date for the purposes of international search only:

          ☐   in the form of an Annex C/ST.25 text file (Rule 13*ter*.1(a)).

          ☐   on paper or in the form of an image file (Rule 13*ter*.1(b) and Administrative Instructions, Section 713).

2.   ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

### INTERNATIONAL SEARCH REPORT
#### Information on patent family members

| | International application No. |
|---|---|
| | **PCT/KR2022/007695** |

| Patent document cited in search report | | | Publication date (day/month/year) | Patent family member(s) | | | Publication date (day/month/year) |
|---|---|---|---|---|---|---|---|
| US | 10822661 | B2 | 03 November 2020 | US | 10801072 | B2 | 13 October 2020 |
| | | | | US | 2017-0233825 | A1 | 17 August 2017 |
| | | | | US | 2017-0275702 | A1 | 28 September 2017 |
| | | | | WO | 2016-036994 | A1 | 10 March 2016 |
| | | | | WO | 2016-036994 | A8 | 10 March 2016 |
| | | | | WO | 2016-037000 | A1 | 10 March 2016 |
| KR | 10-2013-0043104 | A | 29 April 2013 | AU | 2011-223789 | A1 | 20 September 2012 |
| | | | | AU | 2011-237669 | A1 | 01 November 2012 |
| | | | | AU | 2011-237669 | B2 | 08 September 2016 |
| | | | | AU | 2011-291599 | A1 | 07 March 2013 |
| | | | | AU | 2011-291599 | B2 | 10 September 2015 |
| | | | | AU | 2012-220872 | A1 | 12 September 2013 |
| | | | | AU | 2012-267884 | A1 | 23 January 2014 |
| | | | | AU | 2012-271516 | A1 | 23 January 2014 |
| | | | | AU | 2012-294458 | A1 | 27 February 2014 |
| | | | | AU | 2015-268602 | A1 | 07 January 2016 |
| | | | | BR | 112012025593 | A2 | 25 June 2019 |
| | | | | BR | 112013031591 | A2 | 13 December 2016 |
| | | | | BR | 112013032232 | A2 | 20 September 2016 |
| | | | | BR | 112014002975 | A2 | 01 March 2017 |
| | | | | BR | PI0919882 | A2 | 16 February 2016 |
| | | | | BR | PI0921043 | A2 | 07 August 2018 |
| | | | | CA | 2742324 | A1 | 03 June 2010 |
| | | | | CA | 2743211 | A1 | 20 May 2010 |
| | | | | CA | 2791905 | A1 | 09 September 2011 |
| | | | | CA | 2795776 | A1 | 13 October 2011 |
| | | | | CA | 2808417 | A1 | 23 February 2012 |
| | | | | CA | 2827894 | A1 | 30 August 2012 |
| | | | | CA | 2838728 | A1 | 13 December 2012 |
| | | | | CA | 2839530 | A1 | 20 December 2012 |
| | | | | CA | 2844671 | A1 | 14 February 2013 |
| | | | | CN | 102301002 | A | 28 December 2011 |
| | | | | CN | 102308004 | A | 04 January 2012 |
| | | | | CN | 103025890 | A | 03 April 2013 |
| | | | | CN | 103237901 | A | 07 August 2013 |
| | | | | CN | 103237901 | B | 03 August 2016 |
| | | | | CN | 103492590 | A | 01 January 2014 |
| | | | | CN | 103782174 | A | 07 May 2014 |
| | | | | CN | 103797131 | A | 14 May 2014 |
| | | | | CN | 103874770 | A | 18 June 2014 |
| | | | | CN | 107254538 | A | 17 October 2017 |
| | | | | EP | 2350320 | A2 | 03 August 2011 |
| | | | | EP | 2358912 | A2 | 24 August 2011 |
| | | | | EP | 2358912 | B1 | 12 October 2016 |
| | | | | EP | 2542696 | A1 | 09 January 2013 |
| | | | | EP | 2542696 | B1 | 28 September 2016 |
| | | | | EP | 2556172 | A1 | 13 February 2013 |
| | | | | EP | 2606353 | A1 | 26 June 2013 |
| | | | | EP | 2678448 | A1 | 01 January 2014 |
| | | | | EP | 2718721 | A1 | 16 April 2014 |

Form PCT/ISA/210 (patent family annex) (July 2019)

**INTERNATIONAL SEARCH REPORT**
Information on patent family members

International application No.

**PCT/KR2022/007695**

| Patent document cited in search report | Publication date (day/month/year) | Patent family member(s) | | | Publication date (day/month/year) |
|---|---|---|---|---|---|
| | | EP | 2721179 | A2 | 23 April 2014 |
| | | EP | 2730662 | A1 | 14 May 2014 |
| | | EP | 2742154 | A2 | 18 June 2014 |
| | | EP | 3181705 | A1 | 21 June 2017 |
| | | GB | 2463401 | A | 17 March 2010 |
| | | GB | 2463401 | B | 29 January 2014 |
| | | GB | 2465088 | A | 12 May 2010 |
| | | GB | 2465088 | B | 18 July 2012 |
| | | HK | 1142371 | A1 | 03 December 2010 |
| | | IL | 212591 | A | 31 July 2011 |
| | | IL | 212768 | A | 31 July 2011 |
| | | IL | 221719 | A | 29 November 2012 |
| | | IL | 221719 | B | 31 August 2016 |
| | | IL | 222232 | A | 31 December 2012 |
| | | IL | 229862 | A | 27 February 2014 |
| | | IL | 229991 | A | 27 February 2014 |
| | | IL | 230868 | A | 31 March 2014 |
| | | IL | 233023 | A | 31 March 2016 |
| | | IL | 233188 | A | 31 May 2016 |
| | | IL | 244689 | A | 21 April 2016 |
| | | JP | 2012-507300 | A | 29 March 2012 |
| | | JP | 2012-508577 | A | 12 April 2012 |
| | | JP | 2013-521502 | A | 10 June 2013 |
| | | JP | 2013-526852 | A | 27 June 2013 |
| | | JP | 2013-540995 | A | 07 November 2013 |
| | | JP | 2014-507160 | A | 27 March 2014 |
| | | JP | 2014-519340 | A | 14 August 2014 |
| | | JP | 2014-522993 | A | 08 September 2014 |
| | | JP | 2014-526032 | A | 02 October 2014 |
| | | JP | 2016-028572 | A | 03 March 2016 |
| | | JP | 2016-028584 | A | 03 March 2016 |
| | | JP | 2017-167157 | A | 21 September 2017 |
| | | JP | 5808349 | B2 | 10 November 2015 |
| | | KR | 10-2013-0056855 | A | 30 May 2013 |
| | | KR | 10-2014-0067001 | A | 03 June 2014 |
| | | KR | 10-2014-0072014 | A | 12 June 2014 |
| | | KR | 10-2014-0076543 | A | 20 June 2014 |
| | | US | 2010-0113290 | A1 | 06 May 2010 |
| | | US | 2010-0184046 | A1 | 22 July 2010 |
| | | US | 2010-0203529 | A1 | 12 August 2010 |
| | | US | 2011-0151460 | A1 | 23 June 2011 |
| | | US | 2011-0159506 | A1 | 30 June 2011 |
| | | US | 2011-0237450 | A1 | 29 September 2011 |
| | | US | 2013-0005599 | A1 | 03 January 2013 |
| | | US | 2013-0045882 | A1 | 21 February 2013 |
| | | US | 2013-0178383 | A1 | 11 July 2013 |
| | | US | 2013-0184169 | A1 | 18 July 2013 |
| | | US | 2013-0287772 | A1 | 31 October 2013 |
| | | US | 2014-0141986 | A1 | 22 May 2014 |
| | | US | 2014-0148350 | A1 | 29 May 2014 |

Form PCT/ISA/210 (patent family annex) (July 2019)

**INTERNATIONAL SEARCH REPORT**
Information on patent family members

International application No.

**PCT/KR2022/007695**

| Patent document cited in search report | Publication date (day/month/year) | Patent family member(s) | | | Publication date (day/month/year) |
|---|---|---|---|---|---|
| | | US | 2014-0162888 | A1 | 12 June 2014 |
| | | US | 2014-0220580 | A1 | 07 August 2014 |
| | | US | 2014-0228233 | A1 | 14 August 2014 |
| | | US | 2015-0024961 | A1 | 22 January 2015 |
| | | US | 2015-0301055 | A1 | 22 October 2015 |
| | | US | 2016-0041153 | A1 | 11 February 2016 |
| | | US | 2017-0108503 | A1 | 20 April 2017 |
| | | US | 7888035 | B2 | 15 February 2011 |
| | | US | 7897356 | B2 | 01 March 2011 |
| | | US | 8192954 | B2 | 05 June 2012 |
| | | US | 8211653 | B2 | 03 July 2012 |
| | | US | 8278059 | B2 | 02 October 2012 |
| | | US | 9128101 | B2 | 08 September 2015 |
| | | US | 9469876 | B2 | 18 October 2016 |
| | | WO | 2010-056337 | A2 | 20 May 2010 |
| | | WO | 2010-056337 | A3 | 10 September 2010 |
| | | WO | 2010-062706 | A2 | 03 June 2010 |
| | | WO | 2010-062706 | A3 | 02 September 2010 |
| | | WO | 2011-109440 | A1 | 09 September 2011 |
| | | WO | 2011-127219 | A1 | 13 October 2011 |
| | | WO | 2012-024543 | A1 | 23 February 2012 |
| | | WO | 2012-115885 | A1 | 30 August 2012 |
| | | WO | 2012-170711 | A1 | 13 December 2012 |
| | | WO | 2012-174282 | A2 | 20 December 2012 |
| | | WO | 2012-174282 | A3 | 07 February 2013 |
| | | WO | 2013-022995 | A2 | 14 February 2013 |
| | | WO | 2013-022995 | A3 | 04 April 2013 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JEMAL A et al.** *CA Cancer J Clin,* 2010, vol. 60, 277-300
- **HAN, H. ; BEARSS, D. J ; BROWNE, L. W ; CALA-LUCE, R ; NAGLE, R. B. ; VON HOFF, D. D.** Identification of differentially expressed genes in pancreatic cancer cells using cDNA microarray. *Cancer Res,* 2002, vol. 62 (10), 2890-6
- **KWANG HYUCK LEE.** Quantification of DNA as a tumor marker in patients with pancreatic cancer. *Journal of the Korean Society of Gastroenterology,* 2005, vol. 46, 226-32
- **XUE, D et al.** *Journal of Cellular and Molecular Medicine,* 2018, vol. 22 (6), 3223-3237
- **WANG, F. et al.** *Oncotarget,* 2014, vol. 5 (22), 11091-11102
- **BUSSEMAKERS et al.** *Cancer Research,* 1999, vol. 59 (23), 5975-5979
- **MAO, ZUJIE et al.** *Medicine,* 2018, vol. 97, 42
- **KOO, KEVIN M. et al.** *Scientific reports,* 2016, vol. 6 (1), 1-6
- **URBINATI, GIORGIA et al.** *Molecular Therapy-Nucleic Acids,* 2016, vol. 5
- **FREDSØJ. et al.** *Eur. Urol. Focus,* 2018, vol. 4, 825-833
- **URABE, F. et al.** *Clin. Cancer Res.,* 2019, vol. 25, 3016-3025
- **XUEPING ZHANG et al.** *Clin Exp Metastasis,* 2009, vol. 26 (8), 965-79
- **KATI P PORKKA et al.** *Cancer Res,* 2007, vol. 67 (13), 6130-5